# EUROPEAN PATENT APPLICATION

(11) **EP 4 293 039 A1**
(43) Date of publication of application: **20.12.2023**
(21) Application number: 22752767.8
(22) Date of filing: 09.02.2022
(51) Int. Cl.: C07K 14/54, A61K 38/20, A61P 31/12, A61P 35/00, A61P 37/02, C12N 1/21, C12N 5/0783, C12N 5/10, C12N 15/24

(54) **NOVEL HUMAN INTERLEUKIN-18 VARIANT AND USE THEREFOR**

(30) Priority: 10.02.2021 JP 2021019418
(71) Applicant: Nagasaki University, Nagasaki-Shi, Nagasaki 852-8521 (JP); Foundation for Biomedical Research and Innovation at Kobe, Kobe-shi, Hyogo 650-0047 (JP); National Institutes for Quantum Science and Technology, Chiba 263-8555 (JP)
(72) Inventor: TANAKA, Yoshimasa, Nagasaki-shi, Nagasaki 852-8521 (JP); SAKURABA, Shun, Chiba-shi, Chiba 263-8555 (JP)
(74) Representative: Hoffmann Eitle
(86) International application number: PCT/JP2022/005051
(87) International publication number: WO 2022/172944

(57) **Abstract**

The present invention relates to a novel interleukin-18 (IL-18) variant and a pharmaceutical composition using the same. Specifically, the present invention relates to a human IL-18 variant including (i) mutations of cysteine at positions 38, 68, 76, and 127 into serine and (ii) mutations of glutamic acid at position 6 and lysine at position 53 into alanine relative to an amino acid sequence of wild-type human IL-18, wherein the variant further includes (iii) at least one additional mutation, and a pharmaceutical composition containing said human IL-18 variant.

## Description

### Technical Field

### [Related applications]

The present description encompasses the contents described in the description of patent application No. 2021-19418 (filed February 10, 2021), which is the basis of the priority of the present application.

### [Technical Field]

The present invention relates to a novel human interleukin-18 variant. More specifically, it relates to a stable and highly active human interleukin-18 variant.

### Background Art

Interleukin-18 (IL-18) is a cytokine belonging to the IL-1 superfamily discovered as an interferon γ (IFNγ) production inducer (Non Patent Literature 1). IL-18 is produced as an inactive precursor (pro-IL-18) containing a propeptide and is released extracellularly as an active form of IL-18 after cleavage by caspase 1, caspase 4, or the like.

IL-18 is produced in the immune system, mainly by macrophage-based cells, binds to IL-18 receptors expressed on the cell surface of cells in which IFNγ production is induced in the presence of IL-12, facilitates IFNγ production, and enhances Th1-type immune response. Meanwhile, in the absence of IL-12, IL-18 enhances Th2-type immune response. For example, IL-18 acts on NK cells in the presence of IL-2 to induce Th2-type immune response and acts on eosinophils and mast cells in the presence of IL-3 to enhance Th2-type immune response.

Native human IL-18 is unstable and, when recombinantly produced, tends to aggregate to form oligomers and have reduced activity. This is because the cysteine residue exposed to the surface of the IL-18 molecule forms a disulfide bond with a cysteine residue of other IL-18 molecules, thereby oligomerized (Non Patent Literature 2).

Yamamoto et al. report that the substitution of all four cysteines (positions 38, 68, 76, and 127) in the molecule of IL-18 with serine enables the production of stable human IL-18 (Non Patent Literature 2). Okamura et al. report that substituting cysteine (C) at positions 38, 76, or 127 with serine (S) or alanine (A) results in a polypeptide with significantly increased stability (Patent Literature 1). Bam et al. have produced a PEGylated IL-18 variant by substituting cysteine (C) at positions 38 and 68 with serine (S) or aspartic acid (D), and substituting one of the tip residues (positions 78, 121, 157) of the flexible surface loop not involved in the interaction with the IL-18 receptor with cysteine (C) to which PEG is attached (Patent Literature 2).

IL-18 exhibits physiological activity by binding to an IL-18 receptor on a cell membrane. Since IL-18 binding proteins (IL-18BPs) specific for IL-18 are excessively present *in vivo,* most of the secreted IL-18 binds to IL-18BP and rapidly loses its activity.

Kim et al. report, based on predictions from computer models of the complex of IL-18 and IL-18BP, that substitutions of glutamic acid (E) at position 6 and lysine (K) at position 53, which are important for the interaction with IL-18BP, with an uncharged amino acid (such as alanine) significantly improves the activity of human IL-18 (Non Patent Literature 3). Shirakawa et al. report that substitutions of aspartic acid at positions 17, 35, and 132 with alanine result in significant suppression of binding to the receptor (Patent Literature 3). Seatang et al. report that the substitution of glutamic acid (E) at position 6 with lysine (K) and the substitution of threonine (T) at position 63 with lysine (K) lead to a significant increase in activity, and the substitution of methionine at position 33 or 60 with glutamine leads to decrease or loss of activity (Non Patent Literature 4).

However, in some reports so far, the activity of wild-type IL-18 for comparison is significantly low. Thus, the described activity value of the variant compared to the wild-type may be not appropriate, and it needs to be compared and verified with the wild-type having the proper activity.

Zhou et al. have produced an IL-18 variant having reduced interaction with IL-18BP by introducing random substitutions into tyrosine (Y) at position 1, leucine (L) at position 5, lysine (K) at position 8, methionine (M) at position 51, lysine (K) at position 53, serine (S) at position 55, glutamine (Q) at position 56, proline (P) at position 57, glycine (G) at position 59, methionine (M) at position 60, glutamic acid (E) at position 77, glutamine (Q) at position 103, serine (S) at position 105, aspartic acid (D) at position 110, asparagine (N) at position 111, methionine (M) at position 113, valine (V) at position 153, and asparagine (N) at position 155 in wild-type human IL-18, based on predictions from computer models of the complex of IL-18 and IL-18BP (Patent Literature 4, Non Patent Literature 5). However, since the region important for interaction with IL-18BP is also involved in binding to IL-18Rα, modifications of the above positions may lead to decreased activity of IL-18.

### Citation List

### Patent Literature

Patent Literature 1: Japanese Patent Laid-Open No. 10-262686
Patent Literature 2: WO2004/091517
Patent Literature 3: Japanese Patent Laid-Open No. 2005-52021
Patent Literature 4: WO2019/051015

### Non Patent Literature

Non Patent Literature 1: Okamura et al., "Cloning of a new cytokine that induces IFN-gamma production by T cells" Nature. 1995 Nov 2;378(6552):88-91
Non Patent Literature 2: Yamamoto et al., "Generation of highly stable IL-18 based on a ligand-receptor complex structure" Biochem Biophys Res Commun. 2004 Apr 23;317(1):181-186
Non Patent Literature 3: Kim et al., "Site-specific mutations in the mature form of human IL-18 with enhanced biological activity and decreased neutralization by IL-18 binding protein" Proc Natl Acad Sci. 2001 Mar 13;98(6):3304-3309
Non Patent Literature 4: Saetang et al., "Immunologic Function and Molecular Insight of Recombinant Interleukin-18" PLoS One. 2016 Aug 2;11(8):e0160321
Non Patent Literature 5: Zhou et al., "IL-18BP is a secreted immune checkpoint and barrier to IL-18 immunotherapy" Nature. 2020 Jul;583(7817):609-614

### Summary of Invention

### Technical Problem

The activity of IL-18 is influenced by aggregation (oligomerization), binding to IL-18BP, and affinity for IL-18R. An object of the present invention is to provide a stable and highly active novel human IL-18 variant, considering all these influences.

### Solution to Problem

The present inventors have found that the substitution of four cysteines with serine to prevent aggregation of IL-18, and further substitutions of glutamic acid at position 6 and lysine at position 53 with an uncharged amino acid to reduce binding to IL-18BP result in a reduction of IL-18 activity. It has been thought that this is because the interaction of IL-18 and its receptor, IL-18R α-chain, depends on glutamic acid at position 6 and lysine at position 53. The inventors have thus extensively searched for a variant with high structural stability and high activity by introducing a point mutation into a CSEK variant having substitutions of four cysteines with serine and substitutions of glutamic acid at position 6 and lysine at position 53. Further, based on the obtained variants, the inventors have searched a highly active variant with fewer mutations. It should be noted that, unless otherwise specified, the position of each amino acid used herein indicates the position in the amino acid sequence of mature human IL-18 that does not contain a propeptide.

The present invention is based on the above-mentioned study results, and relates to the following items [1] to [22].
[1] A human IL-18 variant comprising an amino acid sequence having substitutions of cysteine at positions 38, 68, 76, and 127 with serine and substitutions of glutamic acid at position 6 and lysine at position 53 with alanine relative to an amino acid sequence of wild-type human IL-18, wherein the variant has at least one additional mutation introduced therein, and has a higher activity than the wild-type human IL-18.
[2] The human IL-18 variant according to [1], wherein the additional mutation is selected from a mutation at positions 3, 34, 38, 51, 72, 112, 119, or 145, and an introduction of a disulfide bond between positions 7 and 50 in the amino acid sequence of the wild-type human IL-18.
[3] The human IL-18 variant according to [1], wherein the additional mutation is selected from G3Y, G3L, A6W, T34P, C38M, M51Y, S72Y, S72F, S72M, S72L, S72W, K112W, K119V, G145N, and S7/50C.
[4] The human IL-18 variant according to any one of [1] to [3], wherein the activity is an activity of enhancing interferon γ production induction. For example, when used at a concentration of 3 ng/ml, the human IL-18 variant of the present invention has a higher activity of enhancing interferon γ production induction than the wild-type.
[5] A human IL-18 variant comprising an amino acid sequence having substitutions of cysteine at positions 38, 68, 76, and 127 with serine and substitutions of glutamic acid at position 6 and lysine at position 53 with alanine relative to an amino acid sequence of wild-type human IL-18, wherein the variant has at least one additional mutation introduced therein, and the additional mutation is any one selected from G3Y, G3L, A6W, T34P, C38M, M51Y, S72Y, S72F, S72M, S72L, S72W, K112W, K119V, G145N, and S7/50C.
[6] The human IL-18 variant according to any one of [1] to [5], wherein the additional mutation is any one selected from G3Y, G3L, C38M, S72Y, S72F, S72M, S72L, and S72W.
[7] The human IL-18 variant according to any one of [1] to [6], wherein the amino acid sequence of the wild-type human IL-18 includes an amino acid sequence represented by SEQ ID NO: 3.
[8] A pharmaceutical composition comprising the human IL-18 variant according to any one of [1] to [7].
[9] The pharmaceutical composition according to [8], for treating a viral disease, a cancer, or an immune disease which are sensitive to IFNγ.
[10] The pharmaceutical composition according to [8] or [9], wherein the pharmaceutical composition is used in combination with at least one selected from the group consisting of an anti-PD-1 antibody, an anti-PD-L1 antibody, an anti-CTLA-4 antibody, and IL-2.
[11] An agent for enhancing T-cell growth induction comprising the human IL-18 variant according to any one of [1] to [7].
   Preferably, the agent for enhancing T-cell growth induction is used in combination with IL-2.
[12] A method for producing a genetically modified T cell, the method comprising a step of inducing a growth of a cell population including T cells in the presence of IL-2 and the human IL-18 variant according to any one of [1] to [7].
   For example, a peripheral blood mononuclear cell population including T cells is isolated from peripheral blood of a subject and stimulated with anti-CD3 and anti-CD28 antibodies, then the growth induction of the population is enhanced with IL-2 and the human IL-18 variant to expand T cells. A gene such as chimeric antigen receptor (CAR) is then introduced into the expanded T cells, and the T cells after gene introduction are further subjected to the enhancement of growth induction with IL-2 and the IL-18 variant to expand T cells.
[13] A human IL-18 variant comprising an amino acid sequence having a substitution of any one amino acid selected from amino acids at positions 3, 38, and 72 with another amino acid relative to an amino acid sequence of wild-type human IL-18.
[14] A human IL-18 variant comprising an amino acid sequence having any one of mutations selected from G3Y, G3L, C38M, S72Y, S72F, and S72M, preferably any one of mutations selected from G3Y, G3L, and C38M, more preferably a mutation of G3L or C38M introduced therein relative to an amino acid sequence of wild-type human IL-18.
[15] A human IL-18 variant comprising an amino acid sequence having substitutions of cysteine at positions 38, 68, 76, and 127 with serine relative to an amino acid sequence of wild-type human IL-18, and having at least one additional mutation introduced therein, wherein the additional mutation is any one selected from G3Y, G3L, C38M, S72Y, S72F, and S72M, preferably any one selected from G3Y, G3L, and C38M, more preferably G3L or C38M.
[16] A human IL-18 variant having a G3L mutation relative to an amino acid sequence of wild-type human IL-18, wherein the human IL-18 variant optionally has an additional mutation of the following 1) or 2):
   1) substitutions of cysteine at positions 38, 68, 76, and 127 with serine; or
   2) substitutions of cysteine at positions 38, 68, 76, and 127 with serine and substitutions of glutamic acid at position 6 and lysine at position 53 with alanine.
[17] A human IL-18 variant having a C38M mutation relative to an amino acid sequence of wild-type human IL-18, wherein the human IL-18 variant optionally has an additional mutation of the following 1) or 2):
   1) substitutions of cysteine at positions 68, 76, and 127 with serine; or
   2) substitutions of cysteine at positions 68, 76, and 127 with serine and substitutions of glutamic acid at position 6 and lysine at position 53 with alanine.
[18] The human IL-18 variant according to any one of [13] to [17], wherein the amino acid sequence of the wild-type human IL-18 includes an amino acid sequence represented by SEQ ID NO: 3.
[19] A pharmaceutical composition comprising the human IL-18 variant according to any one of [13] to [17].
[20] The pharmaceutical composition according to [19], for treating a viral disease, a cancer, or an immune disease which are sensitive to IFNγ.
[21] The pharmaceutical composition according to [19] or [20], wherein the pharmaceutical composition is used in combination with at least one selected from the group consisting of an anti-PD-1 antibody, an anti-PD-L1 antibody, an anti-CTLA-4 antibody, and IL-2.
[22] An agent for enhancing T-cell growth induction comprising the human IL-18 variant according to any one of [13] to [17].

### Advantageous Effects of Invention

The human IL-18 variant of the present invention has high stability and reduced binding to IL-18BP and also has high activity (e.g., an ability of enhancing IFNγ production induction). The human IL-18 variant of the present invention is thus useful for the treatment of cancers and immune diseases based on IL-18 activity.

### Brief Description of Drawings

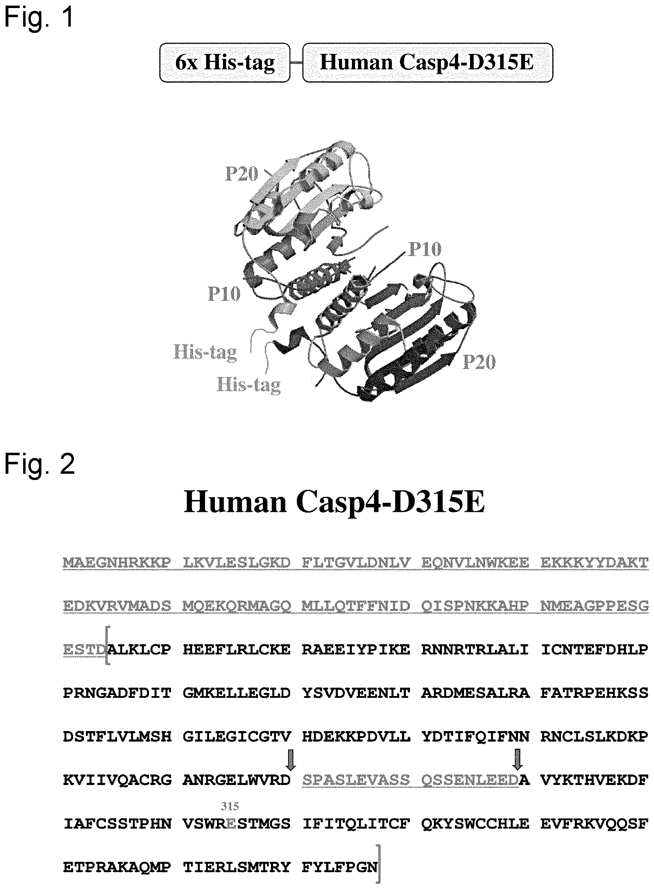

[Figure 1] Figure 1 shows the configuration of a human caspase 4 (D315E) variant forming a dimer. A histidine tag is added to the N-terminal.
[Figure 2] Figure 2 shows the amino acid sequence of a human caspase 4 (D315E) variant precursor. The 104 amino acids first underlined indicate a propeptide. The two arrows indicate the positions of self-digestion. The part shown in [ ] is the amino acid sequence of mature human caspase 4 that does not contain a propeptide.

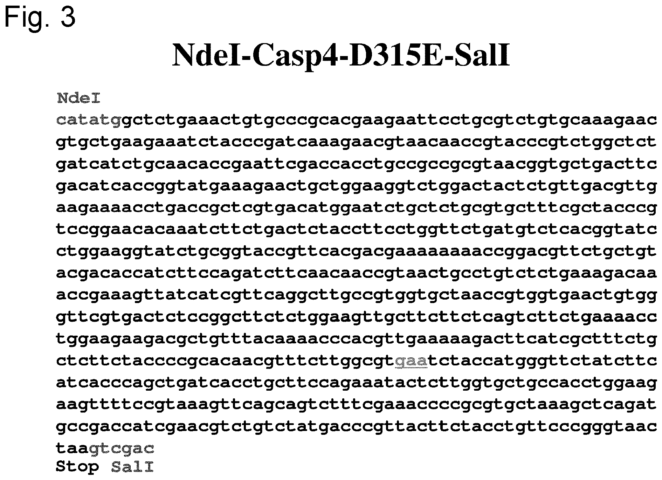

[Figure 3] Figure 3 shows NdeI-Casp4-D315E-SalI in which restriction enzyme recognition sequences are added at both ends of a DNA sequence encoding human caspase 4 (D315E) variant optimized for E. coli. The first catatg indicates an NdeI recognition sequence, the last gtcgc indicates a recognition sequence of SalI, and taa in front of the SalI recognition sequence indicates a stop codon. The underlined gaa corresponds to a mutation introduction site (a site of substitution from aspartic acid to glutamic acid).

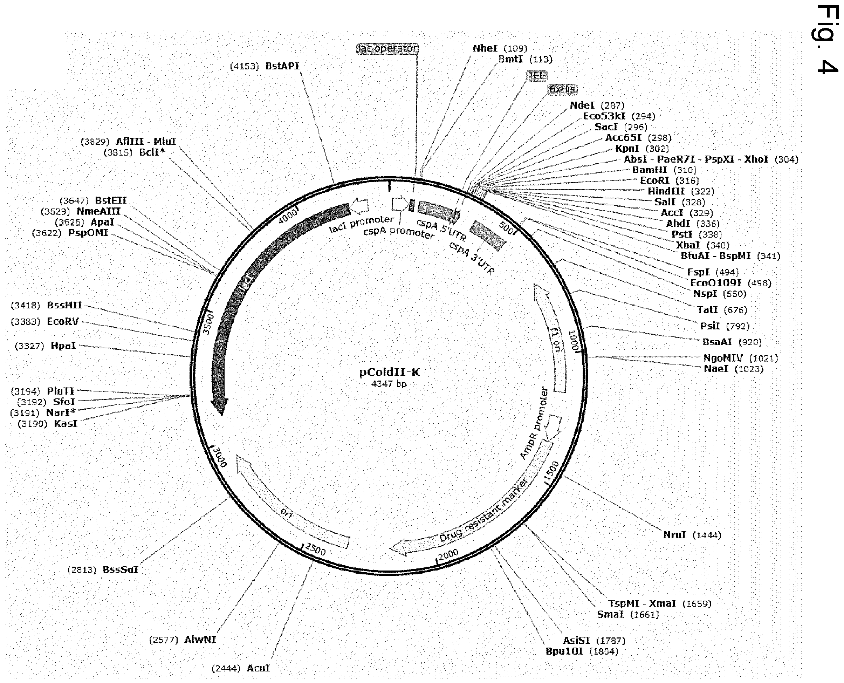

[Figure 4] Figure 4 shows a vector for expressing human caspase 4 (D315E).

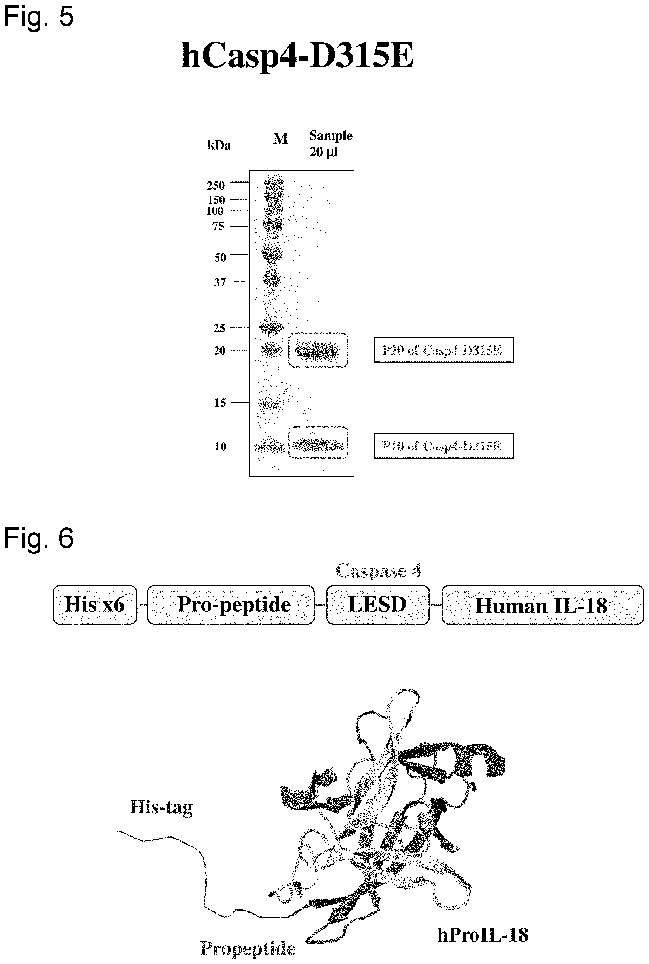

[Figure 5] Figure 5 shows an image of SDS-PAGE electrophoresis results for human caspase 4 (D315E).
[Figure 6] Figure 6 shows the configuration of a human IL-18 precursor (wild-type) for recombinant expression. A histidine tag is added to the N-terminus of the propeptide. The precursor is cleaved by a caspase at aspartic acid (D) on the C-terminal side of the propeptide to produce mature human IL-18.

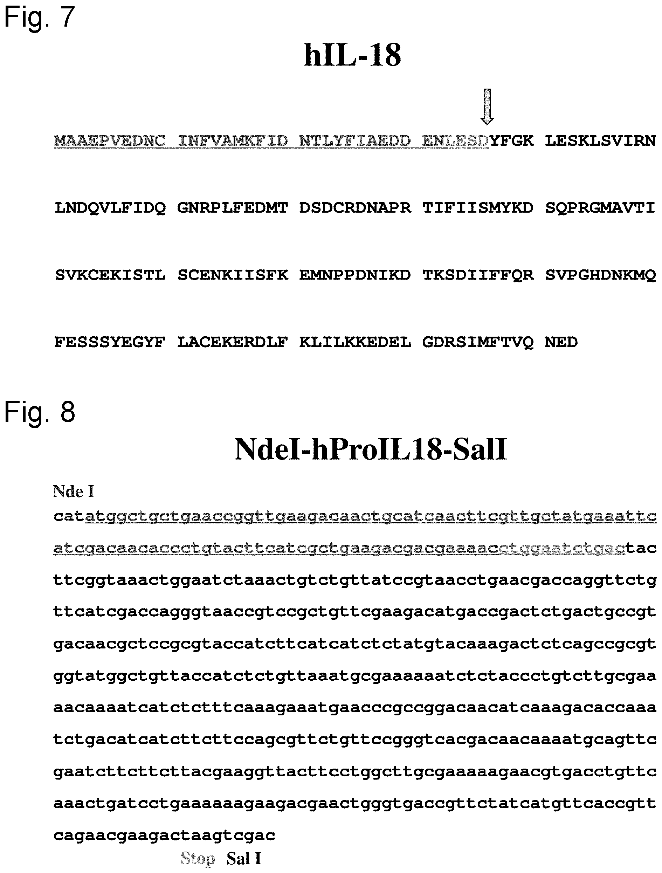

[Figure 7] Figure 7 shows the amino acid sequence of the human IL-18 precursor (wild-type). The 36 amino acids first underlined is a propeptide, LESD at the C-terminal side is a caspase-4 recognition sequence, and the arrow indicates a cleavage site by caspase 4.
[Figure 8] Figure 8 indicates NdeI-hProIL18-SalI in which restriction enzyme recognition sequences are added at both ends of the DNA sequence encoding a human IL-18 precursor (wild-type) optimized for E. coli. The first catatg indicates an NdeI recognition sequence, the last gtcgac indicates a recognition sequence of SalI, and taa in front of the SalI recognition sequence indicates a stop codon. The underlined part corresponds to the propeptide, and ctggaatctgac (positions 100 to 111 of SEQ ID NO: 4) on the C-terminal side of the propeptide corresponds to the caspase-4 recognition sequence.

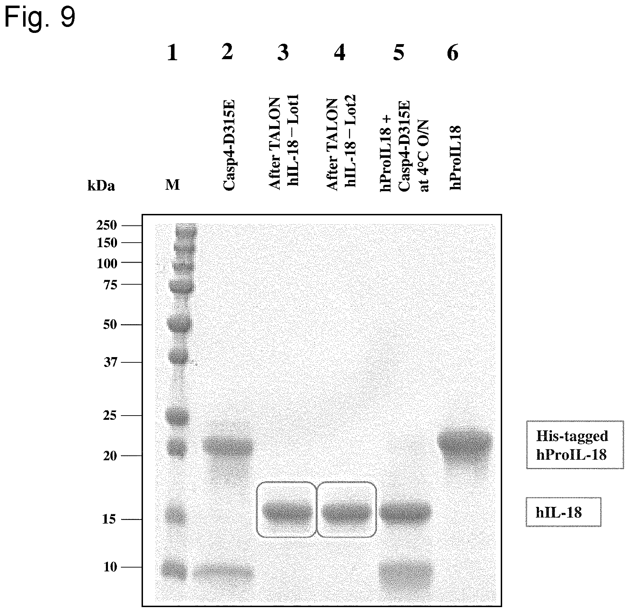

[Figure 9] Figure 9 shows an image of SDS-PAGE electrophoresis results for human IL-18 (wild-type). 1: molecular weight marker, 2: P10 and P20 fragments of Casp4-D315E, 3: mature human IL-18 samples 1, 4: mature human IL-18 sample 2 (for confirmation), 5: one obtained after human IL-18 precursor was treated with Casp4-D315E at 4°C all day and night, and 6: human IL-18 precursor.

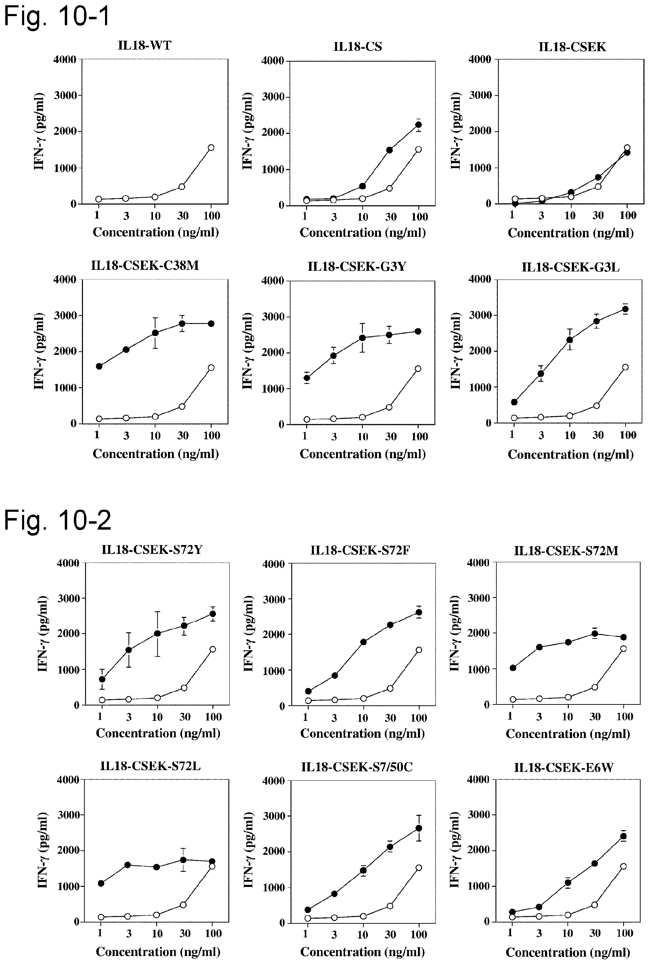

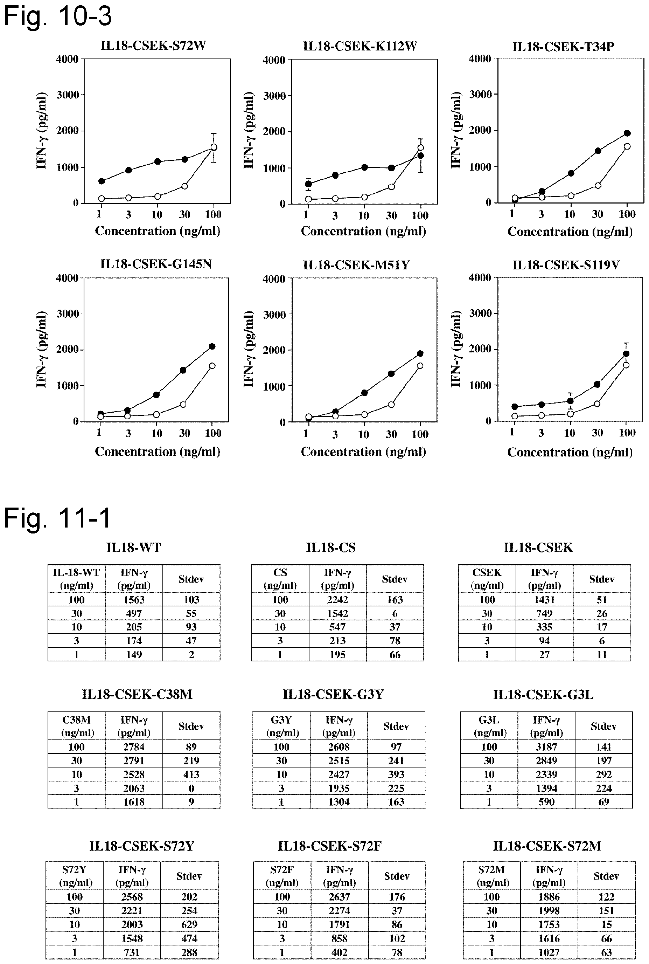

[Figure 10] Figure 10 shows the enhancement of IFNγ production induction by human IL-18 variants. In each graph, the white circle indicates activity of enhancing IFNγ production induction the wild-type, and the black circle indicates the activity of enhancing IFNγ production induction of each variant.

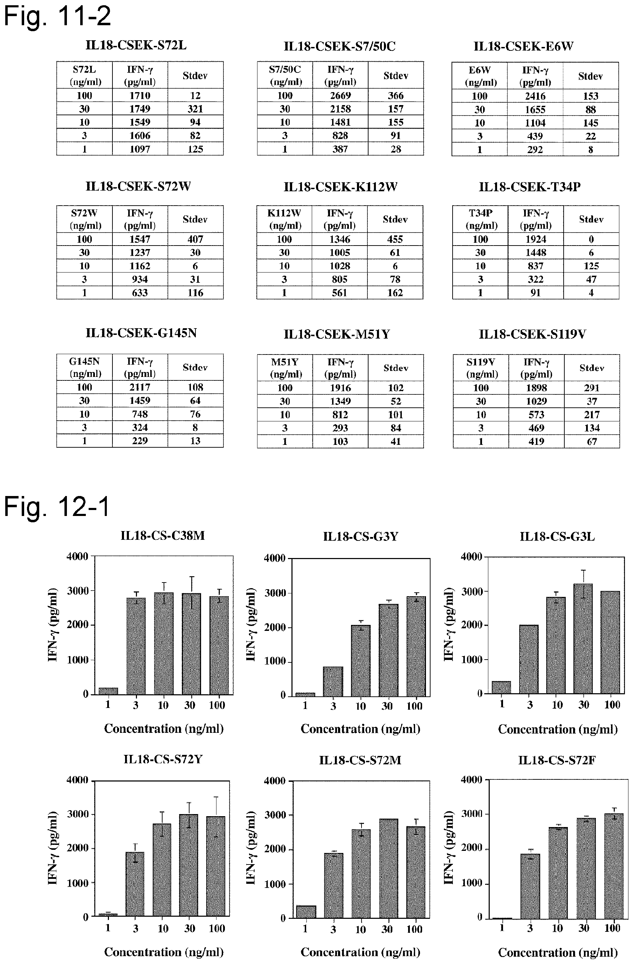

[Figure 11] Figure 11 shows the enhancement of IFNγ production induction by human IL-18 variants. Each table corresponds to each graph in Figure 10.

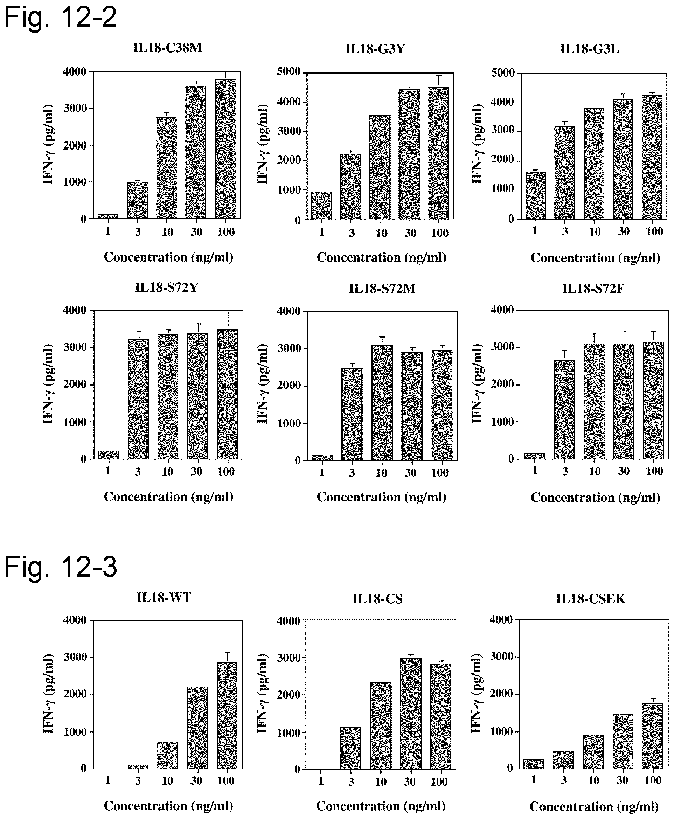

[Figure 12] Figure 12 shows the activity of enhancing IFNγ production induction by IL-18CS variants containing a mutation of G3Y, G3L, C38M, S72Y, S72F, or S72M, IL-18 variants containing a mutation of G3Y, G3L, C38M, S72Y, S72F, or S72M, an IL-18 WT (wild-type), an IL-18CS variant, and an IL-18CSEK variant.

### Description of Embodiments

### 1. Interleukin 18 (IL-18)

### 1.1 Structure and function

Interleukin 18 (IL-18) is a cytokine belonging to the IL-1 superfamily discovered as an IFNγ production inducer. IL-18 is produced as an inactive precursor (pro-IL-18) containing a propeptide and is released as an active form of IL-18 after cleavage of the propeptide by caspase 1, caspase 4, or the like.

The human IL-18 precursor consists of 193 amino acids (24 KDa), and the mature human IL-18 not containing the propeptide consists of 157 amino acids (18 KDa). Each example of the amino acid sequences of a human IL-18 precursor and human IL-18 is described in SEQ ID NO: 2 (Figure 7) and SEQ ID NO: 3, respectively. Human IL-18 precursors and human IL-18 having an amino acid sequence with 80%, 85%, 90%, 95%, 96%, 98%, or 99% or more sequence identity to these sequences can be used as the human IL-18 precursor and human IL-18, respectively, as long as they have human IL-18 activity.

As described above, IL-18 causes inflammation and tissue damage by inducing the production of inflammatory cytokines through the enhancement of IFNγ production induction by IL-12. The effect of this IL-18 on Th1-type immune response is synergistically enhanced by the presence of IL-2. However, under certain conditions, IL-18 can enhance the induction of antiinflammatory cytokines such as IL-4 and IL-13 and also induce Th2-type immune response.

### 1.2 IL-18 binding protein

*In vivo,* IL-18 binding proteins (IL-18BPs) that specifically bind to IL-18 are constantly expressed. Since IL-18BPs have a high affinity of 400 pM and are present in a large excess *in vivo* (at a molar concentration of 20-fold or more relative to IL-18), most of the secreted IL-18 binds to IL-18BP, causing suppression of IL-18 binding to the receptor and loss of IL-18 activity. As such, IL-18BP controls the activity of IL-18 and thereby suppressing the immune response, thus Zhou et al. call IL-18BP as a secretory immune checkpoint (see Zhou et al., supra).

### 1.3 IL-18 Receptor

IL-18 receptors are expressed in various cells, such as NK cells, NKT cells, helper T cells, B cells, neutrophils, and macrophages. The IL-18 receptor binding site consists of an α chain (IL-18Rα) and a β chain (IL-18Rβ). The binding site to IL-18Rα of human IL-18 is reported to possibly be resembled to the binding site to IL-18BP (see Kim et al., supra).

### 2. Human IL-18 variant

The human IL-18 variant of the present invention is a human IL-18 variant having substitutions of cysteine at positions 38, 68, 76, and 127 with serine and substitutions of glutamic acid at position 6 and lysine at position 53 with alanine relative to an amino acid sequence of wild-type human IL-18, and also having at least one additional mutation introduced therein. Hereinafter, the amino acid mutation may be briefly indicated with the amino acid residue and position before the mutation and the amino acid residue after the mutation, for example, a mutation of cysteine (C) at position 38 to serine (S) may be briefly indicated as C38S. The wild-type human IL-18 used may be, for example, one containing the amino acid sequence represented by SEQ ID NO: 3. The wild-type human IL-18 may have an amino acid sequence in which 10, 9, 8, 7, 6, 5, 4, 3, 2, or 1 amino acid is each independently added to one or both ends of the amino acid sequence represented by SEQ ID NO: 3. Preferably, the wild-type human IL-18 has an amino acid sequence consisting of an amino acid sequence represented by SEQ ID NO: 3.

### (1) CS mutation

In the molecule of human IL-18, four cysteines (C) are present at positions 38, 68, 76, and 127, but no disulfide bonds are formed, and all side chains of the cysteine residues are present on the molecular surface. Thus, human IL-18 tends to be oligomerized by the formation of a disulfide bond between molecules with free cysteine residues. This oligomerization suppresses the binding to the receptor and leads to inactivation of human IL-18 (Yamamoto et al., supra). In the human IL-18 variant of the present invention, four cysteines are mutated into serine and thus the oligomerization and thereby the decrease of activity are prevented. Mutations of the four cysteines to serine (C38S, C68S, C76S, C127S) are referred to as "CS mutation".

### (2) EK mutation

Glutamic acid (E) at position 6 and lysine (K) at position 53 of the human IL-18 molecule are important sites for the interaction with IL-18BP (Kim et al. 2001 supra). By substituting glutamic acid at position 6 and lysine at position 53 with amino acids without charge (such as alanine), the binding affinity with IL-18BP can be reduced. Mutations of glutamic acid at position 6 and lysine at position 53 to alanine (A) (E6A, K53A) are hereinafter referred to as "EK mutation".

### (3) CSEK mutation

A variant containing both the CS mutation and the EK mutation is expected to have an improvement in activity by the effect of preventing oligomerization due to the CS mutation and the effect of decreased binding to IL-18BP due to the EK mutation. However, the present inventors have found that the CSEK variant is less active than the CS variant, and have no improvements in activity and rather has equivalent or slightly decreased activity compared to the wild-type. It is considered that this is because glutamic acid (E) at position 6 and lysine (K) at position 53 are also involved in the binding to IL-18Rα, and thus the mutation affects the activity through binding to the receptor. Hereinafter, a mutation containing both the CS mutation and the EK mutation is referred to as a "CSEK mutation", and a human IL-18 variant containing the CSEK mutation is referred to as a "human IL-18CSEK variant".

### (4) Additional mutation

The human IL-18 variant of the present invention has at least one "additional mutation" based on the human IL-18CSEK variant. The "additional mutation" improves the activity of the human IL-18 variant by altering its binding to IL-18Rα without compromising the effects of the CS mutation and the EK mutation in the human IL-18CSEK variant.

The additional mutation includes the following:
(a) a mutation of glycine (G) at position 3: preferably a mutation of glycine (G) at position 3 to tyrosine (Y) (G3Y) and a mutation of glycine (G) at position 3 to leucine (L) (G3L);
(b) a mutation of alanine (A) (glutamic acid (E) in the wild-type sequence) at position 6 to an amino acid having no charge other than alanine: preferably a mutation of alanine (glutamic acid in the wild-type sequence) at position 6 to tryptophan (W) (A6W; E6W based on the wild-type sequence);
(c) a mutation (of threonine) at position 34: preferably a mutation (of threonine) at position 34 to proline (P) (T34P);
(d) a mutation of cysteine (C) at position 38: preferably a mutation of cysteine (C) at position 38 to methionine (M) (C38M);
(e) a mutation of methionine (M) at position 51: preferably a mutation of methionine (M) at position 51 to tyrosine (Y) (M51Y);
(f) a mutation of serine (S) at position 72: preferably a mutation of serine (S) at position 72 to tyrosine (Y) (S72Y), a mutation to phenylalanine (F) (S72F), a mutation to methionine (M) (S72M), a mutation to leucine (L) (S72L), and a mutation to tryptophan (W) (S72W);
(g) a mutation of lysine (K) at position 112: preferably a mutation of lysine (K) at position 112 to tryptophan (W) (K112W);
(h) a mutation of lysine (K) at position 119: preferably a mutation of lysine (K) at position 119 to valine (V) (K119V);
(i) a mutation of glycine (G) at position 145: preferably a mutation of glycine (G) at position 145 to asparagine (N) (G145N);
(j) introduction of a disulfide bond between positions 7 and 50: i.e., mutation of serine (S) at positions 7 and 50 to cysteine (C) (S7/50 C).

More preferably, the additional mutations are G3Y, G3L, C38M, S72Y, S72F, S72M, S72L, S72W, and S7/50C.

Further preferably, the additional mutations are G3Y, G3L, C38M, S72Y, S72F, S72M, S72L, and S72W.

Among the human IL-18CSEK variants described above, those having G3Y, G3L, C38M, S72Y, S72F, and S72M exhibit relatively high activity even when they have no EK and CSEK mutations. That is, a variant having a substitution of an amino acid at position 3, 38, or 72 in the human IL-18CS variant or the human IL-18 wild-type with an amino acid different from the wild-type amino acid, particularly, a variant having a point mutation selected from G3Y, G3L, C38M, S72Y, S72F, and S72M introduced therein, is also encompassed within the scope of the present invention.

### 2.2 Amino acid sequence of human IL-18 variant

The preferred amino acid sequences of a human IL-18 variant of the present invention are represented by SEQ ID NOs: 9, 11, 13, 15, 17, 19, 21, 23, 25, 27, 29, 31, 33, 35, and 37. Variants containing minor modifications that do not alter the structural stability and activity of the human IL-18 variant at sites other than the sites of the CS mutation (C38S, C68S, C76S, C127S), the EK mutation (E6A, K53A), and additional mutations (e.g., G3Y, G3L, A6W, T34P, C38M, M51Y, S72Y, S72F, S72M, S72L, S72W, K112W, K119V, G145N, and S7/50C) described above in these amino acid sequences are also encompassed in the human IL-18 variant of the present invention. The human IL-18 variant containing such minor modifications has 80% or more, preferably 90% or more, more preferably 95% or more, 96% or more, 97% or more, 98% or more, or 99% or more sequence identity to the amino acid sequences represented by SEQ ID NOs: 9, 11, 13, 15, 17, 19, 21, 23, 25, 27, 29, 31, 33, 35, 37, 45, 47, 49, 51, 53, 55, 57, 59, 61, 63, 65, and 67.

### 2.3 DNA sequence encoding human IL-18 variant

DNA sequences encoding human IL-18 variants are optimized for recombinant production as described below, depending on the host used. Examples of the codon-optimized DNA sequence to express in E. coli for a human IL-18 variant having the amino acid sequence described above include those represented by SEQ ID NOs: 10, 12, 14, 16, 18, 20, 22, 24, 26, 28, 30, 32, 34, 36, 38, 46, 48, 50, 52, 54, 56, 58, 60, 62, 64, 66, and 68, respectively. The DNA sequence is appropriately optimized depending on the host used.

### 2.4 IL-18 activity

The human IL-18 variant of the present invention has both stability and improved activity. Examples of the IL-18 activity can include enhancement of IFNγ production induction, enhancement of growth induction of immune effector cells, such as CD8-positive killer αβ T cells, γδ T cells, NK cells, and CD4 CD8-negative killer αβ T cells (NKT cells), in particular, preferably, NK cells, γδ type T cells, and more preferably NK cells, enhanced expression of major histocompatibility antigens such as HLA-DR/HLA-DQ, enhanced expression of CD80/CD86, enhanced expression of CD25, and enhanced expression of ICOS. In the present invention, the phrase "having a higher activity than the wild-type human IL-18" means that any of the activities described above is higher than that of the wild-type.

Preferably, the human IL-18 variant of the present invention has an improved ability (activity) of enhancing IFNγ production induction compared to the wild-type human IL-18. For example, the human IL-18 variant of the present invention has an improved ability (activity) of enhancing IFNγ production induction at a concentration of 3 ng/ml compared to the wild-type.

The human IL-18 variant of the present invention may be modified, for example, prenylated, acetylated, amidated, carboxylated, glycosylated, or PEGylated, depending on the purpose, and such a modified human IL-18 variant is also within the scope of the present invention.

### 3. Production of human IL-18 variant

### 3.1 Codon Optimization

DNA sequences encoding human IL-18 (precursor) are preferably optimized depending on the host used to maximize the expression. Codon optimization can be performed using commercial software such as GeneOptimizer^{®} for codon optimization.

### 3.2 Introduction of mutation

The introduction of mutations into human IL-18 (precursor) can be performed by a total synthesis of DNA. It can also be performed using a commercial site-specific mutation induction system according to the previous report (Yamamoto et al., and Kim et al., supra). Specifically, a mutation of interest can be introduced by providing a pair of primers that sandwich a mutation-introducing site of interest, incorporating the mutation of interest into one of the primers, and amplifying a plasmid containing the template sequence using the pair of primers. The introduction of the mutation of interest can be confirmed by ligating the amplified sequences, cloning with E. coli or the like, and then sequencing.

### 3.3 Expression of variant

The DNA encoding a human IL-18 variant (precursor) containing a mutation of interest is incorporated into an appropriate expression vector and then introduced into a host cell to be expressed.

The host cell used is not particularly limited as long as the cell is capable of expressing human IL-18, and examples thereof include prokaryotic cells such as E. coli, and eukaryotic cells such as yeast, insect cells, and animal cells. Examples of E. coli include E. coli XL1-Blue, E. coli XL2-Blue, E. coli DH1, E. coli MC1000, E. coli KY3276, E. coli W1485, E. coli JM109, E. coli HB101, E. coli No.49, E. coli W3110, E. coli NY49, E. coli DH5α, E. coli Rosetta^{™} (DE3)pLysS, and E. coli Rosetta^{™}2(DE3)pLysS.

The expression vector is not particularly limited as long as it can be expressed in the host. Examples of the vector that can be used in E. coli include pAT153, pACYC184, pBR322, pBR325, pBR327, pBR328, pUC type, pBluescript II, pMTL20, pBS, pGEM, pBEMEX, pUR222, pUCBM, pSP type, pEX type, pCAT type, pT3/T7, pEUK, pMAM, pMSG, pEMBL, pSELECT, pBTrp2, pBTac1, pBTac2, pKK233-2, pSE280, pGEMEX-1, pQE-8, pGEX, pET type, pME18SFL3pGEX-4T-1, pACYC177, pKK338-1, pKC30, pKT279, pFB type, pNO1523, pSVL, pKSV10, pGA482, pNOS, pHSV106, and pCold^{™} series.

Examples of the vector that can be used in yeasts can include YEP13, YEp24, and YCp50. Examples of the vector that can be used in insect cells include pVL1392, pVL1393, and pBlueBacIII. Examples of the vector that can be used in plant cells can include Ti plasmids and tobacco mosaic virus vectors.

Examples of the vector that can be used in animal cells include pcDNAI, pCDM8, pAGE107, pCDM8, pcDNAI/Amp, pcDNA3.1, pREP4, pAGE103, pAGE210, pME18SFL3, and INPEP4.

Since human IL-18 does not have a carbohydrate chain, the human IL-18 variant (precursor) of the present invention can be easily recombinantly expressed using E. coli as a host.

The human IL-18 variant may be fused with a soluble tag sequence, such as a histidine (His) tag, a glutathione S-transferase (GST) tag, or the like for expression to facilitate subsequent purification and detection.

### 3.4 Purification

The human IL-18 variant (precursor) is purified by crushing the cell with ultrasound or a cell wall lysing enzyme, then separating the polypeptide from the cell or cell lysate by filtration, centrifugation, or the like. For purification, the methods well known in the art, such as salt dialysis, dialysis, filtration, concentration, fractional precipitation, gel filtration chromatography, ion exchange chromatography, hydrophobic chromatography, affinity chromatography, chromatofocusing, gel electrophoresis, isoelectric point electrophoresis, and the like can be used.

### Affinity chromatography

When a soluble tag such as a His tag or a GST tag is introduced in advance to the human IL-18 variant (precursor), purification can be easily performed by affinity chromatography.

Because the His tag binds to immobilized metal ions such as nickel, cobalt, and copper under certain conditions, human IL-18 variants expressed as fusion proteins with the His tag can be readily purified using immobilized metal affinity chromatography (IMAC). Examples of IMAC include a nickel column, which is an IMAC using nickel, and a TALON column, which is an IMAC using cobalt.

The GST tag selectively binds to a glutathione resin under normal conditions, thus human IL-18 variants expressed as fusion proteins with the GST tag can be readily purified by chromatography using a glutathione resin.

### Gel filtration

The molecular weight of the human IL-18 variant is 18 kDa. The carrier to be used can be appropriately selected considering the molecular weight of the protein of interest, the scale, and the operating time. The gel filtration chromatography may be used in combination with the affinity chromatography described above.

### 3.5 Cleavage of propeptide

Cleavage of the propeptide is required to generate a mature human IL-18 variant from the human IL-18 variant precursor. For cleavage of the propeptide, caspase 1, caspase 4, caspase 5, or the like can be used. The origin of the caspase used is not particularly limited, but the caspase derived from human is preferred.

Caspases are proteases that play a central role in processes such as apoptosis and inflammation. In mammals, caspases 1 to 14 have been found, and caspases 1, 4, and 5 are all known as inflammatory caspases. Caspases 1, 4, and 5 are all expressed as inactive precursors (procaspases) and they undergo protease processing to become a mature caspase which is composed of heterotetramers consisting of P20 and P10 subunits.

For caspases 1, 4, and 5, those commercially available may be used. Since the amino acid sequence and the DNA sequences encoding thereof are known, caspases 1, 4, and 5 may also be produced by recombinant production according to the conventional method as described for IL-18 variants. In the recombinant production, an appropriate mutation may be introduced at a vulnerable site to prevent self-digestion of human caspase.

In Examples described below, human caspase 4 (D315E) (SEQ ID NO: 40) having a mutation of aspartic acid at position 315 in the amino acid sequence of wild-type human caspase 4 precursor (SEQ ID NO: 39) to glutamic acid was used. This recombinant human caspase 4 variant is also within the scope of the invention.

In addition to caspases, factor Xa can also be used for cleavage of the propeptide of IL-18 (Yamamoto et al. and Kim et al., supra). Factor Xa is a protease of 59 Da composed of two subunits. As factor Xa, commercially available factor Xa derived from bovine plasma can be used. The amino acid sequence of factor Xa and the DNA sequence encoding thereof are also known (GenBank Accession No. NM_001080213.2), thus factor Xa may be produced and used by conventional methods.

### 3.6 Activity measurement

The activity of the human IL-18 variants can be confirmed by comparing any of the enhancement of IFNγ production induction, the enhancement of NK cell growth induction, the enhancement of HLA-DR/DQ expression, the enhancement of CD80/CD86 expression, and the enhancement of CD25 expression to the activity of wild-type IL-18.

The ability of enhancing IFNγ production induction can be confirmed by adding a human IL-18 variant to IFNy-producing cells such as HBL-38 cells, Mo cells, Jurkat cells, HuT78 cells, EL4 cells, L12-R4 cells, KG-1 cells (human acute myelogenous leukemia cells) at about 0.1 ng to 1 µg/ml, preferably about 1 to 100 ng/ml and then culturing them, and measuring the amount of IFNγ in the culture solution by ELISA or the like. The IL-18 variant of the present invention has a higher ability of enhancing IFNγ production induction than the wild-type.

The enhancement of NK cell proliferation induction can be confirmed by adding an IL-2/IL-18 variant to human peripheral blood mononuclear cells that have been cultured after removing CD3-positive cells, and confirming the increase in NK cell-specific markers (e.g., CD56-positive, CD161-negative, CD3-negative) by counting the number of cells.

The change in molecule expressions on the NK cells can be confirmed by flow cytometry or the like. Specifically, it can be confirmed by comparing HLA-DR/DQ and CD80/86 expressed on NK cells cultured with IL-2/IL-18 variants as described above with those expressed on NK cells cultured with IL-2 alone.

Because there is no cross-reactivity between human IL-18 and mouse IL-18 (mouse IL-18 precursor: SEQ ID NO: 43, mature mouse IL-18: SEQ ID NO: 44), it is required to produce a mouse IL-18 variant corresponding to the human IL-18 variant of the present invention for experiments using mice. Since there is a certain degree of homology between the primary structure of human IL-18 and mouse IL-18, a corresponding mouse IL-18 variant can be obtained by introducing the CS mutation into wild-type mouse IL-18, substituting at sites important for interaction with IL-18BP, and further introducing additional mutations according to the method described above.

### 4. Pharmaceutical composition

The human IL-18 variant of the present invention has a high activity of enhancing IFNγ production induction or the like and is thus useful for a pharmaceutical composition for treating or preventing a viral disease, a cancer, and an immune disease that are sensitive to IFNγ.

The pharmaceutical composition containing the human IL-18 variant of the present invention may contain a pharmacologically acceptable carrier or an additive. Examples of such a carrier and an additive include, but are not limited to, an excipient, a binder, a lubricant, a solvent, a disintegrant, a dissolution aid, a suspending agent, an emulsifying agent, an isotonic agent, a stabilizer, an analgesic agent, a preservative, an antioxidant, a taste masking agent, a coloring agent, a buffer, a fluidity enhancing agent, and the like. Other conventional carriers and additives can be used as appropriate.

Examples of the excipient include saccharides such as lactose, glucose, and D-mannitol; organic excipients such as starches and celluloses including crystalline cellulose; and inorganic excipients such as calcium carbonate and kaolin.

Examples of the binder include pregelatinized starch, gelatin, gum arabic, methyl cellulose, carboxymethyl cellulose, sodium carboxymethyl cellulose, crystalline cellulose, D-mannitol, trehalose, hydroxypropyl cellulose, hydroxypropyl methyl cellulose, polyvinylpyrrolidone, and polyvinyl alcohol.

Examples of the lubricant include fatty acid salts such as stearic acid and stearate, talc, and silicates.

Examples of the solvent include purified water, physiological saline, and phosphate buffer.

Examples of the disintegrant include low-substituted hydroxypropyl cellulose, chemically modified cellulose, and starches.

Examples of the dissolution aid include polyethylene glycol, propylene glycol, trehalose, benzyl benzoate, ethanol, sodium carbonate, sodium citrate, sodium salicylate, and sodium acetate.

Examples of the suspending agent or the emulsifying agent include sodium lauryl sulfate, gum arabic, gelatin, lecithin, glyceryl monostearate, polyvinyl alcohol, polyvinylpyrrolidone, celluloses such as sodium carboxymethylcellulose, polysorbates, and polyoxyethylene cured castor oil.

Examples of the isotonic agent include sodium chloride, potassium chloride, saccharides, glycerin, and urea.

Examples of the stabilizer include polyethylene glycol, sodium dextran sulfate, and other amino acids.

Examples of the analgesic agent include glucose, calcium gluconate, and procaine hydrochloride.

Examples of the preservative include paraoxybenzoic acid esters, chlorobutanol, benzyl alcohol, phenethyl alcohol, dehydroacetic acid, and sorbic acid.

Examples of the antioxidant include water-soluble antioxidants such as ascorbic acid, cysteine hydrochloride, sodium bisulfate, sodium metabisulfite, and sodium sulfite; lipid-soluble antioxidants such as ascorbyl palmitate, butylated hydroxyanisole (BHA), butylated hydroxytoluene (BHT), lecithin, propylgalate, and alpha-tocopherol; and metal chelating agents such as citric acid, ethylenediaminetetraacetic acid (EDTA), sorbitol, tartaric acid, and phosphoric acid.

Examples of the taste and smell masking agent include sweeteners and flavors commonly used in the pharmaceutical field. Examples of the coloring agent include colorants commonly used in the pharmaceutical field.

The administration route of the pharmaceutical composition of the present invention is not particularly limited, and may be oral or parenteral administration. Specific examples of the parenteral administration include injection administration, nasal administration, pulmonary administration, and transdermal administration. Examples of the injection administration include intravenous injection, intramuscular injection, intraperitoneal injection, subcutaneous injection, intramedullary injection, intrathecal injection, and intradermal injection. The administration method can be appropriately selected depending on the age and symptoms of the patient.

The pharmaceutical composition of the present invention is administered to a subject in a therapeutically effective amount. The "therapeutically effective amount" refers to an amount of a therapeutic agent that is useful for alleviating a selected condition. The therapeutically effective amount is appropriately determined by the purpose of use, the administration route, the age and symptoms of the patient, and the like. When administered to a human, the therapeutically effective amount is, for example, at least 0.001 mg/kg, preferably 0.01 mg/kg, more preferably 0.1 mg/kg, further preferably 1 mg/kg, and at most 100 mg/kg, preferably 50 mg/kg, more preferably 20 mg/kg, further preferably 10 mg/kg in terms of the human IL-18 variant per patient. For example, the therapeutically effective amount is, for example, 0.001 to 100 mg/kg, preferably 0.01 to 50 mg/kg, more preferably 0.1 to 20 mg/kg, further preferably 1 to 10 mg/kg in terms of the human IL-18 variant per patient.

Examples of the cancer to which the pharmaceutical composition of the present invention can be applied include, but are not limited to, leukemia such as acute myelogenous leukemia, chronic myelogenous leukemia, acute lymphoblastic leukemia, and chronic lymphoblastic leukemia, malignant lymphoma such as Hodgkin lymphoma and non-Hodgkin lymphoma, multiple myeloma, myelodysplastic syndrome, head and neck cancer, tongue cancer, pharyngeal cancer, oral cancer, esophageal cancer, esophageal adenocarcinoma, gastric cancer, colorectal cancer, colon cancer, rectal cancer, liver cancer, gallbladder and bile duct cancer, biliary tract cancer, pancreatic cancer, thyroid cancer, lung cancer such as non-small cell lung cancer and small cell lung cancer, breast cancer, ovarian cancer, cervical cancer, uterine cancer, endometrial cancer, vaginal cancer, vulvar cancer, kidney cancer, adenocarcinoma, urothelial cancer, prostate cancer, testicular tumor, bone and soft tissue sarcoma, skin cancer such as uveal malignant melanoma, malignant melanoma, and Merkel cell carcinoma, neuroblastoma, glioblastoma, brain tumor, and pleural mesothelioma.

Examples of the viral disease to which the pharmaceutical composition of the present invention can be applied include, but are not limited to, viral diseases relating to human immunodeficiency virus (HIV), human hepatitis A virus (HAV), human hepatitis B virus (HBV), human hepatitis C virus (HCV), herpes simplex virus (HSV), human papillomavirus (HPV), and the like, particularly a viral disease with an immune deficiency.

Examples of the immune disease to which the pharmaceutical composition of the present invention can be applied include, but are not limited to, rheumatoid arthritis, multiple sclerosis, Crohn's disease, ulcerative colitis, nephritis, psoriasis, asthma, pernicious anemia, hyperthyroidism, autoimmune hemolytic anemia, myasthenia gravis, systemic erythema lupus, Addison's disease, Hodgkin's disease, and AIDS.

The pharmaceutical composition of the present invention may be used in combination with other drugs or treatment methods, as long as the purpose of the present invention is not impaired.

For the treatment of a cancer, the present pharmaceutical composition can be used in appropriate combination with the following: a molecular targeted drug, e.g., a monoclonal antibody including an immune checkpoint inhibitor (rituximab, trastuzumab, gemtuzumab, cetuximab, bevacizumab, panitumumab, ofatumumab, ipilimumab, brentuximab, pertuzumab, obinutuzumab, dinutuximab, nivolumab, pembrolizumab, blinatumomab, ramucirumab, necitumumab, erotuzumab, daratumumab, mogamulizumab, inotuzumab, olaratumab, durvalumab, avelumab, atezolizumab, alemtuzumab, ibritumomab), a tyrosine kinase inhibitor (imatinib, gefitinib, erlotinib, sunitinib, sorafenib, dasatinib, lapatinib, nilotinib, pazopanib, vandetanib, afatinib, bostinib, crizotinib, axitinib, ruxolitinib, regorafenib, ponatinib, cabozantinib, ibrutinib, ceritinib, lenvatinib, nintedanib, osimertinib, alectinib, brigatinib), an mTOR kinase inhibitor (temsirolimus, everolimus), other kinase inhibitors (vemurafenib, dabrafenib, trametinib, palbociclib, cobimetinib, midstaurin, idelalisib), a proteasome inhibitor (bortezomib, carfilzomib, ixazomib), a retinoic acid receptor agonist (tretinoin, alitretinoin, bexarotene, tamibarotene), an SMO antagonist (bismodedib), a VEGF inhibitor (aflibercept), a PARP inhibitor (olaparib, nilaparib); hormone and a hormone antagonist (fluoxymesterone, flutamide, nirtamide, bicalutamide, enzalutamide, ethinyl estradiol, tamoxifen, tremiphene, fluvestrant, luprolerin, goserelin, tryptorelin, anastrozole, letrozole, exemestane, prednisone, octreotide, medroxyprogesterone); an immunofunctional adjuvant therapy, e.g., interferon (IFN-α, IFN-β, IFN-γ, IFN-α2b), interleukin (aldesleukin, oprelbekin, teceleukin, denileukin, diftitox), a colony stimulating factor (filgrastim, sargramostim, lenoglastim, pegfilgrastim), a hematopoietic stem cell mobilizer (plerixafor), a nonspecific immunomodulator (thalidomide, lenalidomide, pomalidomide); an alkylating agent, an antimetabolite, an antibiotic, a microtubule polymer stabilizer (paclitaxel, docetaxel, cabazitaxel), a division inhibitor, a topoisomerase I inhibitor, a topoisomerase II inhibitor, and the like.

In particular, since combination effects of wild-type IL-18 and IL-2 (texeloikin), wild-type IL-18 and an immune checkpoint inhibitor such as an anti-PD-1 antibody (nivolumab, pembrolizumab, cemiplimab, spartalizumab), an anti-PD-L1 antibody (atezolizumab, avelumab, durvalumab), and an anti-CTLA-4 antibody (ipilimumab, tremelimumab) have been reported (Japanese Patent Laid-Open No. 2004-315381, WO2016/021720), a combination effect with the human IL-18 variant of the present invention can also be expected.

For the treatment of a viral disease, the present pharmaceutical composition can be used in appropriate combination with the following: a nucleic acid-based reverse transcriptase inhibitor such as zidovudine, lamivudine, abacavir, tenofovir, or emtricitabine; a non-nucleic acid-based reverse transcriptase inhibitor, such as nevirapine, efavirenz, etrabin, rilpivirine, or dravirin, a protease inhibitor such as nelfinavir, ritonavir, lopinavir, atazanavir, or darnavir; an integrase inhibitor such as laltegravir, erbidegravir, doltegravir; an entry inhibitor such as maraviroc; or a compounding agent comprising one or two or more of these.

For the treatment of an immune disease, the present pharmaceutical composition can be used in appropriate combination with the following: NSAIDs such as celecoxib and sodium roxoprofen; DMARDs such as busilamine, salazosulfapyridine, methotrexate, myzolibine, or tacrolimus; a biological agent such as infliximab, adalimumab, ethanercept, avatasept, tocilizumab, golimumab, sertolizumab begol, lemicade, or human recombinant IL-12; a steroid drug such as prednisolone; or a JAK inhibitor such as tofacitinib.

For formulation, the human IL-18 variant can be complexed with a water-soluble polymer such as PEG (WO2004/091517).

### 6. Other uses

### 6.1 Growth inducer, stimulant

It is known that γδ-type T cells stimulated and cultured with PTA/IL-2/IL-18 have a higher number of cells after culture than γδ-type T cells stimulated and cultured with PTA/IL-2. In addition, in immune effector cells such as αβ-type T cells, γδ-type T cells, or NK cells, and killer cells, the addition of IL-18 improves the expression of major histocompatibility antigens such as HLA-DR and HLA-DQ, CD80, CD86 (positive parastimulatory molecule), CD25 (IL-2 receptor), ICOS (positive parastimulatory molecule), and the like and leads to excellent immune effector cells with antigen presentation ability.

The human IL-18 variants of the present invention have a high activity to activate and induce growth of such NK cells, CD8-positive killer αβ-type T cells, γδ-type T cells, and thus can be used as growth inducers or stimulators of NK cells, CD8-positive killer αβ-type T cells, and γδ-type T cells. For example, by adding the human IL-18 variant of the present invention together with IL-2 to a cell population containing T cells, the T cells can be expanded.

### 6.2 Preparation of genetically modified T cells

The human IL-18 variant of the present invention can be used for T cell expansion in genetically modified T cell therapy such as CAR-T therapy. Specifically, the genetically modified T cells can be efficiently prepared by isolating a peripheral blood mononuclear cell population containing T cells from the peripheral blood of a subject, stimulating it with anti-CD3 and anti-CD28 antibodies, then inducing its growth with IL-2 and a human IL-18 variant to expand the T cells, then introducing a gene, and further inducing a growth of the T cells after the gene transfer with IL-2 and an IL-18 variant to expand the T cells.

For example, by adding IL-18 in addition to IL-2 and inducing a growth in a CAR-T therapy utilizing T cells expressing chimeric antigen receptor (CAR), where peripheral blood mononuclear cells are stimulated with anti-CD3 and anti-CD28 antibodies and IL-2 is added to induce a growth to expand the T cells, the T cells can be expanded more efficiently.

In addition to the above, the human IL-18 variant of the present invention can also be used as a stimulant or a growth inducer in diagnostic and evaluation methods using activation and growth of NK cells, CD8-positive killer αβ-type T cells, and γδ-type T cells as indicators.

### Examples

Hereinafter, the present invention is described in detail with Examples, but the present invention is not limited to these Examples.

### Example 1: Preparation of human caspase 4 variant (hCasp4-D315E)

Human caspase 4 for cleavage of pro-IL-18 (IL-18 precursor) was prepared for recombinant production of human IL-18. Human caspase 4 is a heterodimer composed of two subunits P10 and P20. In this Example, a variant (hCasp4-D315E (FIG. 2, SEQ ID NO: 40) in which aspartic acid (D) at position 315 was substituted with glutamic acid (E) in the amino acid sequence of the wild-type human caspase 4 precursor (SEQ ID NO: 39) was prepared to prevent self-digestion of P10.

### (1) Production of His-tagged hCasp4-D315E expression vector

A DNA sequence encoding hCasp4-D315E without containing a propeptide was optimized for expression in E. coli (SEQ ID NO: 41). To the N-terminus and C-terminus of the optimized sequence, recognition sequences of restriction enzymes NdeI and SalI were added respectively to prepare NdeI-hCasp4-D315E-SalI (Figure 3, SEQ ID NO: 42). NdeI-hCasp4-D315E-SalI was incorporated into a multicloning site of pUCIDT-Amp plasmid. The resulting plasmid was used to transform E. coli DH5a (TOYOBO CO., LTD.). The colonies were then screened with ampicillin-containing LB medium (LB/Amp), and pUCIDT-NdeI-hCasp4-D315E-SalI was extracted from the colonies.

NdeI-hCasp4-D315E-SalI cut from pUCID-Amp-NdeI-hCasp4-D315E-SalI was incorporated into the multicloning site of the pCold^{™} II vector (Takara Bio Inc.) (Figure 4). The pCold^{™} II vector expresses a protein of interest as a soluble protein fused to a His tag when cooled to 15°C. The resulting plasmid was used to transform E. coli DH5a (TOYOBO CO., LTD.). The colonies were then screened with LB medium containing a selective agent, and a vector containing hCasp4-D315E was extracted from the colonies.

### (2) Expression of His-tagged hCasp4-D315E

E. coli BL21(DE3) pLysS was transformed with the above vector, and the generated colonies were transferred to a tube in which 10 ml of LB medium containing a selective agent was placed, and cultured at 37°C for 10 hours. 10 ml of the culture solution was inoculated into a culture medium (1 L, x 4) and cultured at 37°C overnight. 50 ml of the culture solution was inoculated into a culture medium (1 L, x 4) and cultured at 37°C for 8 hours (OD600 nM: 1.641). 1 ml of 1M IPTG was added to the culture solution so that the final concentration was 1 mM to prepare an E. coli suspension. The E. coli suspension was cultured at 15°C for an additional 20 hours to induce expression of soluble hCasp4-D315E (His-tagged hCasp4-D315E). The suspension after culture was centrifuged (7,000 rpm, 10 min, 4°C), the supernatant was then discarded, and the E. coli pellets were collected and stored frozen at -30°C.

### (3) Purification of His-tagged hCasp4-D315E

Frozen E. coli pellets were resuspended in 20 mL of 20 mM sodium phosphate buffer (pH 7.4, containing 300 mM NaCl). The suspension was sonicated (20 sec, 5x) and centrifuged (7,000 rpm, 30 min, 4°C), and the supernatant was transferred to a 50 ml conical tube. 100 µl of DNAse1 was added into the tube, then the mixture was centrifuged (10,000 rpm, 30 min, 4°C), and the supernatant was transferred to a new 50 ml conical tube. The suspension was filtered through a 0.22 mM filter to obtain a sample containing His-tagged hCasp4-D315E. The obtained samples were subjected to TALON Superflow column chromatography (3 ml bed volume) to purify His-tagged hCasp4-D315E. The purified His-tagged hCasp4-D315E was further purified with Superdex 200 pg HiLoad 26/600 columns and confirmed by SDS-PAGE (Figure 5).

### Example 2: Preparation of native human IL-18

Native human IL-18 is expressed as a precursor containing a propeptide (SEQ ID NO: 2), and under the action of caspase, it becomes a mature hIL-18 (SEQ ID NO: 3) having activity (Figure 6, Figure 7). In this Example, hProIL-18 was expressed in E. coli and treated with hCasp4-D315E prepared in Example 1 to produce hIL-18.

### (1) Preparation of His-tagged hProIL18 expression vector

A DNA sequence encoding human IL-18 containing a propeptide (SEQ ID NO: 1, http://www.ncbi.nlm.nih.gov/nuccore/BC007461.1) was optimized for expression in E. coli. To the N- and C-terminus of the optimized sequence, the recognition sequences of restriction enzymes NdeI and SalI were added, respectively, to produce NdeIhProIL18-SalI (Figure 8, SEQ ID NO: 4). NdeI-hProIL18-SalI was incorporated into the multicloning site of pUCIDT-Amp plasmid. The resulting plasmid was used to transform E. coli DH5a (TOYOBO CO., LTD.). The colonies were then screened with LB/Amp, and pUCIDT-NdeI-hProIL18-SalI was extracted from the colonies.

NdeI-hProIL18-SalI cut from pUCIDT-NdeI-hProIL18-SalI was incorporated into the multicloning site of the pCold^{™} II vector. The resulting plasmid was used to transform E. coli DH5a (TOYOBO CO., LTD.). The colonies were then screened with an LB medium containing a selective agent, and a vector containing hProIL18 was extracted from the colonies.

### (2) Expression of His-tagged hProIL18

E. coli Rosetta (DE3) pLysS (Merck KGaA) was transformed with the above vector, and the generated colonies were transferred to a tube in which 10 ml of LB medium containing a selective agent was placed, and cultured at 37°C for 7 hours. 10 ml of the culture solution was inoculated into a culture medium (1 L, x 4) and cultured at 37°C overnight. 100 ml of the culture solution was inoculated into a culture medium (1 L, x 4) and cultured at 37°C for 5 hours and 30 minutes. 1 ml of 1 M IPTG was added to the culture solution so that the final concentration was 1 mM to prepare an E. coli suspension (OD600 nM: 2.102). The E. coli suspension was cultures at 15°C for an additional 40 hours and 30 minutes to induce expression of soluble hProIL18 (His-tagged hProIL18). The suspension after culture was centrifuged (7,000 rpm, 10 min, 4°C), the supernatant was then discarded, and the E. coli pellets were collected and stored frozen at -30°C.

### (3) Purification of His-tagged hProIL18

Frozen E. coli pellets were resuspended in 20 mL of 20 mM sodium phosphate buffer (pH 7.4, containing 300 mM NaCl). The suspension was sonicated (20 sec, 5x) and centrifuged (7,000 rpm, 30 min, 4°C), and the supernatant was transferred to a 50 ml conical tube. 200 µl of DNAse1 was added into the tube, then the mixture was centrifuged (33,000 rpm, 30 min, 4°C), and the supernatant was transferred to a new 50 ml conical tube. The suspension was filtered through a 0.22 mM filter to obtain a sample containing His-tagged hProIL18. The obtained sample was subjected to TALON Superflow column (3 ml bed volume) to purify His-tagged hProIL18. The purified His-tagged hProIL18 was further purified with a Superdex 200 pg HiLoad 26/600 column. The purified hProIL18 was treated with hCasp4-D315E, and hIL18 was confirmed by SDS-PAGE (Figure 9).

### Example 3: Preparation of human IL-18-CS

hIL-18-CS (SEQ ID NO: 5) in which all cysteine residues at positions 38, 68, 76, and 127 in human IL-18 were substituted with alanine was prepared.

### (1) Preparation of His- and GST-tagged hProIL18-CS expression vector

A DNA sequence encoding human IL-18-CS containing a propeptide was optimized for expression in E. coli. To the N-terminus and C-terminus of the optimized sequence, the recognition sequences of restriction enzymes NdeI and SalI were added, respectively to produce NdeI-hProIL18-CS-SalI (SEQ ID NO: 6). NdeI-hProIL18-CS-SalI was incorporated into the multicloning site of pUCIDT-Amp plasmid. The resulting plasmid was used to transform E. coli DH5a (TOYOBO CO., LTD.). The colonies were then screened with LB/Amp, and pUCIDT-NdeI-hProIL18-CS-SalI was extracted from the colonies.

NdeI-hProIL18-CS-SalI cut from pUCIDT-NdeI-hProIL18-CS-SalI was incorporated into the multicloning site of the pCold^{™} GST vector. The pCold^{™} GST vector (Takara Bio Inc.) expresses a protein of interest as a soluble protein fused to a His-GST tag when cooled to 15°C. The resulting plasmid was used to transform E. coli DH5a (TOYOBO CO., LTD.). The colonies were then screened with an LB media containing a selective agent, and a vector containing hProIL18-CS was extracted from the colonies.

### (2) Expression of His- and GST-tagged hProIL18-CS

E. coli Rosetta (DE3) pLysS (Merck KGaA) was transformed with the above vector, and the generated colonies were transferred to a tube in which 10 ml of LB medium containing a selective agent was placed, and cultured at 37°C for 24 hours. 10 ml of the culture solution was inoculated into a culture medium (1 L, x 4) and cultured at 37°C overnight. 50 ml of the culture solution was inoculated into a culture medium (1 L, x 4), and the cells were cultured at 37°C for 8 hours. 1 ml of 1 M IPTG was added to the culture solution so that the final concentration was 1 mM to prepare an E. coli suspension (OD600 nM: 2.690). The E. coli suspension was cultured at 15°C for an additional 40 hours to induce expression of soluble hProIL18-CS (His- and GST-tagged hProIL18-CS). The suspension after culture was centrifuged (7,000 rpm, 10 min, 4°C), the supernatant was then discarded, and the E. coli pellets were collected and stored frozen at -30°C.

### (3) Purification of His- and GST-tagged hProIL18-CS

Frozen E. coli pellets were resuspended in 20 mL of 20 mM sodium phosphate buffer (pH 7.4, containing 300 mM NaCl). The suspension was sonicated (20 sec, 5x) and centrifuged (7,000 rpm, 30 min, 4°C), and the supernatant was transferred to a 50 ml conical tube. 200 µl of DNAse1 was added into the tube, then the mixture was centrifuged (10,000 rpm, 30 min, 4°C), and the supernatant was transferred to a new 50 ml conical tube. The suspension was filtered through a 0.22 mM filter to obtain a sample containing His- and GST-tagged hProIL18-CS. The obtained sample was subjected to a TALON Superflow column (3 ml bed volume) to purify His- and GST-tagged hProIL18-CS. The purified His- and GST-tagged hProIL18-CS was further purified with a Superdex 200 pg HiLoad 26/600 column. The purified hProIL18-CS was treated with hCasp4-D315E, and hIL18-CS was confirmed by SDS-PAGE.

### Example 4: Preparation of human IL-18-CSEK

In human IL-18-CS prepared in Example 3, a glutamic acid residue at position 6 was substituted with alanine (E6A) and a lysine residue at position 53 was substituted with alanine (K53A) to prepare hIL-18-CSEK (SEQ ID NO: 7). The glutamic acid residue at position 6 and lysine at position 53 in human IL-18 are charged amino acids important for interaction with IL-18BP, and it is known that, by removing the charges at this moiety, the affinity with IL-18BP is reduced (Kim et al. 2001, supra).

### (1) Production of His- and GST-tagged hProIL18-CSEK expression vector

A DNA sequence encoding human IL-18-CSEK containing a propeptide was optimized for expression in E. coli. To the N-terminus and C-terminus of the optimized sequence, the recognition sequences of restriction enzymes NdeI and SalI were added, respectively, to produce NdeI-hProIL18-CSEK-SalI (SEQ ID NO: 8). NdeI-hProIL18-CSEK-SalI was incorporated into the multicloning site of pUCIDT-Amp plasmid. The resulting plasmid was used to transform E. coli DH5a (TOYOBO CO., LTD.). The colonies were then screened with LB/Amp, and pUCIDT-NdeI-hProIL18-CSEK-SalI was extracted from the colonies.

NdeI-hProIL18-CSEK-SalI cut from pUCIDT-NdeI-hProIL18-CSEK-SalI was incorporated into the multicloning site of the pCold^{™} GST vector. The resulting plasmid was used to transform E. coli DH5a (TOYOBO CO., LTD.). The colonies were then screened with an LB media containing a selective agent, and a vector containing hProIL18-CSEK was extracted from the colonies.

### (2) Expression of His- and GST-tagged hProIL18-CSEK

E. coli Rosetta (DE3) pLysS (Merck KGaA) was transformed with the above vector, and the generated colonies were transferred to a tube in which 10 ml of LB medium containing a selective agent was placed, and cultured at 37°C for 48 hours. 10 ml of the culture solution was inoculated into a culture medium (1 L, x 4) and cultured at 37°C overnight. 50 ml of the culture solution was inoculated into a culture medium (1 L, x 4), and the cells were cultured at 37°C for 11 hours. To the culture solution, 1 ml of 1 M IPTG was added so that the final concentration was 1 mM, and an E. coli suspension (OD600 nM: 1.894) was prepared. The E. coli suspension was cultured at 15°C for an additional 37 hours to induce expression of soluble hProIL18-CSEK (His- and GST-tagged hProIL18-CSEK). The suspension after culture was centrifuged (7,000 rpm, 10 min, 4°C), the supernatant was then discarded, and the E. coli pellets were collected and stored frozen at -30°C.

### (3) Purification of His- and GST-tagged hProIL18-CSEK

Frozen E. coli pellets were resuspended in 20 ml of 20 mM sodium phosphate buffer (pH 7.4, containing 300 mM NaCl). The suspension was sonicated (20 sec, 5x) and centrifuged (7,000 rpm, 30 min, 4°C), and the supernatant was transferred to a 50 ml conical tube. 200 µl of DNAse1 was added into the tube, then the mixture was centrifuged (10,000 rpm, 30 min, 4°C), and the supernatant was transferred to a new 50 ml conical tube. The suspension was filtered through a 0.22 mM filter to obtain a sample containing His-tagged hProIL18-CSEK. The obtained sample was subjected to a TALON Superflow column (3 ml bed volume) to purify His- and GST-tagged hProIL18-CSEK. The purified His- and GST-tagged hProIL18-CSEK was further purified with a Superdex 200 pg HiLoad 26/600 column. The purified hProIL18-CSEK was treated with hCasp4-D315E, and hIL18-CSEK was confirmed by SDS-PAGE.

### Example 5: Production of various variants based on human IL-18-CSEK

### A. Preliminary analysis

### 1. In silico point mutation scanning

The activity of hIL-18CSEK is lower than that of wild-type hIL-18. It was believed that this is because the structural stability of the protein itself is impaired due to the introduced mutations. Thus, comprehensive introduction of one residue mutation for all amino acids was performed on the computer, and the relative stability of IL18 folding expressed by the difference in Gibbs free energy change (ΔΔG) was calculated with a supercomputer (H. Park et al. 2016). When the protein is predicted to be stabilized, the value of ΔΔG is negative, so this value can be used to perform a quantitative evaluation. Candidates with a value of ΔΔG expected to be negative at this point and candidates of a mutation expected to be stabilized by sequence analysis were subjected to higher accurate ΔΔG prediction (L. Wang et al. 2013). Then, point mutations that reduce ΔΔG without affecting the binding of IL-18CSEK to the IL-18Rα/β protein were searched.

### 2. Introduction of S-S bond

Based on Dombkowski 2003 & Craig and Dombkowski 2013, a new S-S bond was introduced into IL-18 to investigate whether thermal stability could be improved. Based on structural information (Tsutsumi et al. 2014; Protein Data Bank for structure ID: 3W04), we predicted the structural stability when an S-S bond was introduced from the relative positions of Cα-Cβ-Cβ-Cα, and searched a residue pair with low structural distortion energy, that is, one in which an S-S bond is introduced without great disturbance of the structure. Then, as a site into which an S-S bond can be introduced in the same chain with a structural distortion energy below the reference of 2.20 kcal/mol, S7C-S50C (2.05 kcal/mol (chain A), 1.78 kcal/mol (chain B)) was identified.

### B. Introduction of point mutation

Mutations found in *in silico* point mutation scanning were introduced into human IL-18-CSEK and various variants based on human IL-18-CSEK were prepared.

### B-1. Preparation of human IL-18-CSEK-G3Y

In the human IL-18-CSEK prepared in Example 4, a further substitution of the glycine residue at position 3 with tyrosine was made to produce hIL-18-CSEK-G3Y (SEQ ID NO: 9).

### (1) Production of hProIL18-CSEK-G3Y expression vector

A DNA sequence encoding human IL-18-CSEK-C38M containing a propeptide was optimized for expression in E. coli. To the N-terminus and C-terminus of the optimized sequence, the recognition sequences of restriction enzymes NdeI and SalI were added, respectively, to produce NdeI-hProIL18-CSEK-G3Y-SalI (SEQ ID NO: 10). NdeI-hProIL18-CSEK-G3Y-SalI was incorporated into the multicloning site of pUCIDT-Amp plasmid. The resulting plasmid was used to transform E. coli DH5a (TOYOBO CO., LTD.). The colonies were then screened with LB/Amp medium, and pUCIDT-NdeI-hProIL18-CSEK-G3Y-SalI was extracted from the colonies.

NdeI-hProIL18-CSEK-G3Y-SalI cut from pUCIDT-NdeI-hProIL18-CSEK-G3Y-SalI was incorporated into the multicloning site of the pCold^{™} II vector. The resulting plasmid was used to transform E. coli DH5a (TOYOBO CO., LTD.). The colonies were then screened with an LB media containing a selective agent, and a vector containing hProIL18-CSEK was extracted from the colonies.

### (2) Expression of His-tagged hProIL18-CSEK-G3Y

E. coli Rosetta (DE3) pLysS (Merck KGaA) was transformed with the above vector, and the generated colonies were transferred to a tube in which 10 ml of LB medium containing a selective agent was placed, and cultured at 37°C for 7 hours and 30 minutes. 10 ml of the culture solution was inoculated into a culture medium (1 L, x 4) and cultured at 37°C overnight. 100 ml of the culture solution was inoculated into a culture medium (1 L, x 4), and the cells were cultured at 37°C for 7 hours. 1 ml of 1 M IPTG was added to the culture solution so that the final concentration was 1 mM to prepare an E. coli suspension (OD600 nM: 2.295). The E. coli suspension was cultured at 15°C for an additional 40 hours to induce expression of soluble hProIL18-CSEK-G3Y (His-tagged hProIL18-CSEK-G3Y). The suspension after culture was centrifuged (7,000 rpm, 10 min, 4°C), the supernatant was then discarded, and the E. coli pellets were collected and stored frozen at -30°C.

### (3) Purification of His-tagged hProIL18-CSEK-G3Y

Frozen E. coli pellets were resuspended in 20 ml of 20 mM sodium phosphate buffer (pH 7.4, containing 300 mM NaCl). The suspension was sonicated (20 sec, 5x) and centrifuged (7,000 rpm, 30 min, 4°C), and the supernatant was transferred to a 50 ml conical tube. 200 µl of DNAse1 was added to the tube, and the mixture was centrifuged (10,000 rpm, 1 h, 4°C), and the supernatant was transferred to a new 50 ml conical tube. The suspension was filtered through a 0.22 mM filter to obtain a sample containing His-tagged hProIL18-CSEK-G3Y. The obtained sample was subjected to TALON Superflow column (3 ml bed volume) to purify His-tagged hProIL18-CSEK-G3Y. The purified His- and GST-tagged hProIL18-CSEK-G3Y was further purified with a Superdex 200 pg HiLoad 26/600 column. The purified hProIL18-CSEK-G3Y was treated with hCasp4-D315E, and hIL18-CSEK-G3Y was confirmed by SDS-PAGE.

### B-2. Preparation of Human IL-18-CSEK-G3L

In the human IL-18-CSEK prepared in Example 4, a further substitution of the glycine residue at position 3 with tyrosine was made to produce hIL-18-CSEK-G3L (SEQ ID NO: 11).

The DNA sequence encoding human IL-18-CSEK-G3L containing a propeptide was optimized for expression in E. coli according to B-1. To the N-terminus and C-terminus of the optimized sequence, the recognition sequences of restriction enzymes NdeI and SalI were added, respectively, to produce NdeI-hProIL18-CSEK-G3L-SalI (SEQ ID NO: 12), which was then expressed as a soluble protein with E. coli. The protein was purified by TALON column and Superdex 200 pg column, and then treated with hCasp4-D315E to produce hIL18-CSEK-G3L.

### B-3. Preparation of Human IL-18-CSEK-A6W

In the human IL-18-CSEK prepared in Example 4, a further substitution of the glutamic acid residue at position 6 with tryptophan was made to produce IL-18-CSEK-A6W (SEQ ID NO: 13).

The DNA sequence encoding human IL-18-CSEK-A6W containing a propeptide was optimized for expression in E. coli according to B-1. To the N-terminus and C-terminus of the optimized sequence, the recognition sequences of restriction enzymes NdeI and SalI were added, respectively, to produce NdeI-hProIL18-CSEK-A6W-SalI (SEQ ID NO: 14), which was then expressed as a soluble protein with E. coli. The protein was purified by TALON column and Superdex 200 pg column, and then treated with hCasp4-D315E to produce hIL18-CSEK-A6W.

### B-4. Preparation of human IL-18-CSEK-T34P

In the human IL-18-CSEK prepared in Example 4, a further substitution of the thymine residue at position 34 with proline was made to produce IL-18-CSEK-T34P (SEQ ID NO: 15).

The DNA sequence encoding human IL-18-CSEK-T34P containing a propeptide was optimized for expression in E. coli according to B-1. To the N-terminus and C-terminus of the optimized sequence, the recognition sequences of restriction enzymes NdeI and SalI were added, respectively, to produce NdeI-hProIL18CSEK-T34P-SalI (SEQ ID NO: 16), which was then expressed as a soluble protein with E. coli. The protein was purified by TALON column and Superdex 200 pg column, and then treated with hCasp4-D315E to produce hIL18-CSEK-T34P.

### B-5. Preparation of human IL-18-CSEK-C38M

In the human IL-18-CSEK prepared in Example 4, a further substitution of the cysteine residue at position 38 with methionine was made to produce IL-18-CSEK-C38M (SEQ ID NO: 17).

The DNA sequence encoding human IL-18-CSEK-T38M containing a propeptide was optimized for expression in E. coli according to B-1. To the N-terminus and C-terminus of the optimized sequence, the recognition sequences of restriction enzymes NdeI and SalI were added, respectively, to produce NdeI-hProIL18CSEK-C38M-SalI (SEQ ID NO: 18), which was then expressed as a soluble protein with E. coli. The protein was purified by TALON column and Superdex 200 pg column, and then treated with hCasp4-D315E to produce hIL18-CSEK-C38M.

### B-6. Preparation of Human IL-18-CSEK-M51Y

In the human IL-18-CSEK prepared in Example 4, a further substitution of the methionine residue at position 51 with tyrosine was made to produce hIL-18-CSEK-M51Y (SEQ ID NO: 19).

The DNA sequence encoding human IL-18-CSEK-M51Y containing a propeptide was optimized for expression in E. coli according to B-1. To the N-terminus and C-terminus of the optimized sequence, the recognition sequences of restriction enzymes NdeI and SalI were added, respectively, to produce NdeI-hProIL18CSEK-M51Y-SalI (SEQ ID NO: 20), which was then expressed as a soluble protein with E. coli. The protein was purified by TALON column and Superdex 200 pg column, and then treated with hCasp4-D315E to produce hIL18-CSEK-M51Y.

### B-7. Preparation of human IL-18-CSEK-S72Y

In the human IL-18-CSEK prepared in Example 4, a further substitution of the serine residue at position 72 with tyrosine was made to produce hIL-18-CSEK-S72Y (SEQ ID NO: 21).

The DNA sequence encoding human IL-18-CSEK-S72Y containing a propeptide was optimized for expression in E. coli according to B-1 except that the pCold^{™} GST vector (see Example 3) was used instead of the pCold^{™} II vector. To the N-terminus and C-terminus of the optimized sequence, the recognition sequences of restriction enzymes NdeI and SalI were added, respectively, to produce NdeI-hProIL18CSEK-S72Y-SalI (SEQ ID NO: 22), which was then expressed as a soluble protein with E. coli. The protein was purified by TALON column and Superdex 200 pg column, and then treated with hCasp4-D315E to produce hIL18-CSEK-S72Y.

### B-8. Preparation of human IL-18-CSEK-S72F

In the human IL-18-CSEK prepared in Example 4, a further substitution of the serine residue at position 72 with phenylalanine was made to produce hIL-18-CSEK-S72F (SEQ ID NO: 23) .

The DNA sequence encoding human IL-18-CSEK-S72F containing a propeptide was optimized for expression in E. coli according to B-1 except that the pCold^{™} GST vector (see Example 3) was used instead of the pCold^{™} II vector. To the N-terminus and C-terminus of the optimized sequence, the recognition sequences of restriction enzymes NdeI and SalI were added, respectively, to produce NdeI-hProIL18CSEK-S72F-SalI (SEQ ID NO: 24), which was then expressed as a soluble protein with E. coli. The protein was purified by TALON column and Superdex 200 pg column, and then treated with hCasp4-D315E to produce hIL18-CSEK-S72F.

### B-9. Preparation of human IL-18-CSEK-S72M

In the human IL-18-CSEK prepared in Example 4, a further substitution of the serine residue at position 72 with methionine was made to produce hIL-18-CSEK-S72M (SEQ ID NO: 25).

The DNA sequence encoding human IL-18-CSEK-S72M containing a propeptide was optimized for expression in E. coli according to B-1 except that the pCold^{™} GST vector (see Example 3) was used instead of the pCold^{™} II vector. To the N-terminus and C-terminus of the optimized sequence, the recognition sequences of restriction enzymes NdeI and SalI were added, respectively, to produce NdeI-hProIL18CSEK-S72M-SalI (SEQ ID NO: 26), which was then expressed as a soluble protein with E. coli. The protein was purified by TALON column and Superdex 200 pg column, and then treated with hCasp4-D315E to produce hIL18-CSEK-S72M.

### B-10. Preparation of human IL-18-CSEK-S72L

In the human IL-18-CSEK prepared in Example 4, a further substitution of the serine residue at position 72 with leucine was made to produce hIL-18-CSEK-S72L (SEQ ID NO: 27).

The DNA sequence encoding human IL-18-CSEK-S72L containing a propeptide was optimized for expression in E. coli according to B-1 except that the pCold^{™} GST vector (see Example 3) was used instead of the pCold^{™} II vector. To the N-terminus and C-terminus of the optimized sequence, the recognition sequences of restriction enzymes NdeI and SalI were added, respectively, to produce a vector containing NdeI-hProIL18CSEK-S72L-SalI (SEQ ID NO: 28), which was then expressed as a soluble protein with E. coli. The protein was purified by TALON column and Superdex 200 pg column, and then treated with hCasp4-D315E to produce hIL18-CSEK-S72L.

### B-11. Preparation of human IL-18-CSEK-S72W

In the human IL-18-CSEK prepared in Example 4, a further substitution of the serine residue at position 72 with tryptophan was made to produce hIL-18-CSEK-S72W (SEQ ID NO: 29).

The DNA sequence encoding human IL-18-CSEK-S72W containing a propeptide was optimized for expression in E. coli according to B-1 except that the pCold^{™} GST vector (see Example 3) was used instead of the pCold^{™} II vector. To the N-terminus and C-terminus of the optimized sequence, the recognition sequences of restriction enzymes NdeI and SalI were added, respectively, to produce a vector containing NdeI-hProIL18CSEK-S72W-SalI (SEQ ID NO: 30), which was then expressed as a soluble protein with E. coli. The protein was purified by TALON column and Superdex 200 pg column, and then treated with hCasp4-D315E to produce hIL18-CSEK-S72W.

### B-12. Preparation of human IL-18-CSEK-K112W

In the human IL-18-CSEK prepared in Example 4, a further substitution of the lysine residue at position 112 with tryptophan was made to produce hIL-18-CSEK-K112W (SEQ ID NO: 31).

The DNA sequence encoding human IL-18-CSEK-K112W containing a propeptide was optimized for expression in E. coli according to B-1 except that the pCold^{™}GST vector (see Example 3) was used instead of the pCold^{™} II vector. To the N-terminus and C-terminus of the optimized sequence, the recognition sequences of restriction enzymes NdeI and SalI were added, respectively, to produce a vector containing NdeI-hProIL18CSEK-K112W-SalI (SEQ ID NO: 32), which was then expressed as a soluble protein with E. coli. The protein was purified by TALON column and Superdex 200 pg column, and then treated with hCasp4-D315E to produce hIL18-CSEK-K112W.

### B-13. Preparation of human IL-18-CSEK-K119V

In the human IL-18-CSEK prepared in Example 4, a further substitution of the lysine residue at position 119 with valine was made to produce hIL-18-CSEK-K119V (SEQ ID NO: 33).

The DNA sequence encoding human IL-18-CSEK-K119V containing a propeptide was optimized for expression in E. coli according to B-1 except that the pCold^{™} GST vector (see Example 3) was used instead of the pCold^{™} II vector. To the N-terminus and C-terminus of the optimized sequence, the recognition sequences of restriction enzymes NdeI and SalI were added, respectively, to produce a vector containing NdeI-hProIL18CSEK-K119V-SalI (SEQ ID NO: 34), which was then expressed as a soluble protein with E. coli. The protein was purified by TALON column and Superdex 200 pg column, and then treated with hCasp4-D315E to produce hIL18-CSEK-K119V.

### B-14. Preparation of human IL-18-CSEK-G145N

In the human IL-18-CSEK prepared in Example 4, a further substitution of the guanine residue at position 145 with asparagine was made to produce hIL-18-CSEK-G145N (SEQ ID NO: 35).

The DNA sequence encoding human IL-18-CSEK-G145N containing a propeptide was optimized for expression in E. coli according to B-1. To the N-terminus and C-terminus of the optimized sequence, the recognition sequences of restriction enzymes NdeI and SalI were added, respectively, to produce a vector containing NdeI-hProIL18CSEK-G145N-SalI (SEQ ID NO: 36), which was then expressed as a soluble protein with E. coli. The protein was purified by TALON column and Superdex 200 pg column, and then treated with hCasp4-D315E to produce hIL18-CSEK-G145N.

### B-15. Preparation of human IL-18-CSEK-S7/50C

In the human IL-18-CSEK prepared in Example 4, further substitutions of the serine residues at positions 7 and 50 both with cysteine were made to produce hIL-18-CSEK-S7/50C (SEQ ID NO: 37) .

The DNA sequence encoding human IL-18-CSEK-S7/50C containing a propeptide was optimized for expression in E. coli according to B-1. To the N-terminus and C-terminus of the optimized sequence, the recognition sequences of restriction enzymes NdeI and SalI were added, respectively, to produce a vector containing NdeI-hProIL18CSEK-S7/50C-SalI (SEQ ID NO: 38), which was then expressed as a soluble protein with E. coli. The protein was purified by TALON column and Superdex 200 pg column, and then treated with hCasp4-D315E to produce hIL18-CSEK-S7/50C.

### Test Example 1: Measurement 1 of activity of variant - aggregation activity

Frozen KG-1 cells were thawed in 12 ml of RPMI 1640/10% FCS medium. The tube was subjected to centrifugation (1,700 rpm, 5 min, 4°C), and the supernatant was aspirated, then cell pellets were dispersed and re-suspended in 10 ml of RPMI 1640/10% FCS medium (number of cells: 2.00 × 10⁶ cells/ml, total 10 ml). The tube was subjected again to centrifugation (1,700 rpm, 5 min, 4°C) and the supernatant was aspirated, then the cell pellets were dispersed and re-suspended in 10 ml of RPMI 1640/10% FCS medium to obtain 3 × 10⁵/100 ml of KG-1 cell suspensions. The KG-1 cell suspensions (100 ml each) were placed in wells of a 96-well plate.

PBS was added to the IL-18 variant to produce a 50 µg/ml stock solution. To 12 ml of this stock solution, 988 ml of RPMI 1640/10% FCS medium was added to prepare a dilution solution (600 ng/ml). As shown in the table below, this dilution solution was serially diluted with RPMI 1640/10% FCS medium to prepare serially diluted samples (1) to (6). Serially diluted samples were added to each well of the 96-well plate.

**[Table 1]**

| IL-18 dilution solution | RPMI1640/10% FCS medium | Concentration | Final concentration | |
|---|---|---|---|---|
| 400 µl | 800 µl | 200 ng/ml | 100 ng/ml | (1) |
| 300 µl | 700 µl | 60 ng/ml | 30 ng/ml | (2) |
| 400 µl | 800 µl | 20 ng/ml | 10 ng/ml | (3) |
| 300 µl | 700 µl | 6 ng/ml | 3 ng/ml | (4) |
| 400 µl | 800 µl | 2 ng/ml | 1 ng/ml | (5) |
| - | 2,000 µl | 0 ng/ml | 0 ng/ml | (6) |

### Results:

Cells were microscopically observed at 24 and 48 hours after the start of culture. As a result, active IL-18 variants showed concentration-dependent cell aggregation. This concentration-dependency of the aggregation was consistent with the concentration-dependency of IFNy production measured by ELISA (Test Example 2).

### Test Example 2: IFN-y production enhancing activity (1)

A microcentrifuge tube containing 100 µl of human IFN-γ capture antibodies (100 µg/ml) was thawed and the antibodies were diluted with 10 ml of PBS to 1 µg/ml. The diluted capture antibodies (100 µl each) were placed into all wells of the 96-well ELISA plate (Nunc) and the plate was placed at 4°C.

The capture antibodies were removed and the wells were washed four times with PBS/0.05% Tween-20. PBS/0.05% Tween-20 was discarded and a blocking buffer (PBS/1% BSA, 300 ml each) was added to the 96-well ELISA plate, then the plate was sealed and left at room temperature for at least 1 hour. The blocking buffer was discarded and the wells were washed 4 times with PBS/0.05% Tween-20. The 96-well plate containing the KG-1 cell supernatants was thawed, and the supernatants (100 ml each) were added to the ELISA plate.

As shown in the table below, serially diluted solutions of standard human IFNy in PBS/0.05% Tween 80/0.1% BSA were prepared. Serially diluted standard human IFNy (100 µl each) was placed in the 96-well ELISA plate and the plate was left at room temperature for at least 2 hours.

**[Table 2]**

| IFN-γ dilution solution | Diluent (PBS/0.05% Tween 80/0.1%BSA) | Final concentration | |
|---|---|---|---|
| 2.4 µl (1 µg/ml) | 797.6 µl | 3000 pg/ml | (1) |
| 400 µl (3000 pg/ml) | 400 µl | 1500 pg/ml | (2) |
| 400 µl (1500 pg/ml) | 400 µl | 750 pg/ml | (3) |
| 400 µl (750 pg/ml) | 400 µl | 375 pg/ml | (4) |
| 400 µl (375 pg/ml) | 400 µl | 187.5 pg/ml | (5) |
| 400 µl (187.5 pg/ml) | 400 µl | 93.75 pg/ml | (6) |
| 400 µl (93.75 pg/ml) | 400 µl | 46.875 pg/ml | (7) |
| - | 400 µl | 0 pg/ml | (8) |

Samples were discarded and the wells were washed 4 times with 300 ml PBS/0.05% Tween-20. The microcentrifuge tube containing 100 ml of human IFN-y detection antibodies (100 µg/ml) were thawed and the antibodies were diluted with 10 ml of dilution solution to a concentration of 1 µg/ml. The diluted detection antibodies (100 µl each) were added to the 96-well ELISA plate (Nunc), and the plate was left at room temperature for 1 hour. The detection antibodies were aspirated and the wells were washed four times with 300 µl of PBS/0.05% Tween-20. The microcentrifuge tube containing 5.5 µl of avidin-HRP conjugate was thawed and the avidin-HRP conjugate was diluted with 11 ml of diluent. The avidin-HRP conjugates (100 µl each) were dispensed into the 96-well ELISA plate placed at room temperature for 30 minutes. The avidin-HRP conjugate solution was discarded and the wells were washed four times with PBS/0.05% Tween-20. ABTS liquid substrates (100 µl each) were added to the plate and the plate was incubated at room temperature for 5 minutes. ABTS stop solution (1% SDS in DW) was added to the plate, and color generation was monitored with a 405 nm plate reader. The amount of human IFN-y was calculated based on the standard curve. The results are shown in Figures 10 and 11.

### Results:

The IL-18CS variant, in which all four cysteine residues were substituted for serine, had significantly higher activity compared to the wild-type IL-18. Meanwhile, the IL-18CSEK variant, into which the EK substitution has been further introduced to attenuate binding to the IL-18 binding protein, had a reduced binding ability to the IL-18 receptor and had significantly lower activity compared to the IL-18CS variant, and higher activity compared to the wild-type IL-18. Among the IL-18 variants in which one additional amino acid substitution has been introduced into the IL-18CSEK variant, variants having C38M, G3Y, G3L, S72Y, S72F, S72M, S72L, E6W, S72W, K112W, T34P, G145N, M51Y, or S119V showed higher activity than wild-type IL-18. In addition, the S7/50C variant in which S7 and S50 were substituted for C to artificially introduce an S-S bond also had higher activity than the wild-type IL-18.

### Test Example 3: IFN-y production enhancing activity (2)

Among mutations showing relatively high activity in Test Example 2, the mutations G3Y, G3L, C38M, S72Y, S72F, and S72M each were introduced into the IL-18CS variants and the wild-type according to Example 5 to produce IL-18CS variant-based variants and wild-type-based variants. According to Test Example 2, activities of enhancing IFN-y production induction of each of the obtained variants, IL-18 (wild-type: WT), the IL-18CS variant, and the IL-18CSEK variant were measured. The results are shown in Figure 12.

Both the IL-18CS variant-based variants and the wild-type-based variants into which the above point mutation has been added showed higher activity of enhancing IFN-y production induction than the wild-type IL-18, the IL-18CSEK variant, and the IL-18CS variant. G3L and C38M mutations each exhibited a particularly good activity of enhancing IFN-y production induction.

The amino acid sequences of the human IL-18 variants (mutation introduction sites are underlined) are described below in Table 3, and the DNA sequences of the human IL-18 variants (mutation introduction sites are enclosed) optimized for recombinant expression in E. coli are described in Table 4.

**[Table 3]**

| **Example #** | **Amino acid sequence of hIL-18 variant (without propeptide)** |
|---|---|
| **3: CS SEQ ID NO:5** | YFGKLESKLSVIRNLNDQVLFIDQGNRPLFEDMTDSDSRDNAPRTIFIIS MYKDSQPRGMAVTISVKSEKISTLSSENKIISFKEMNPPDNIKDTKSDIIF FQRSVPGHDNKMQFESSSYEGYFLASEKERDLFKLILKKEDELGDRSI MFTVQNED |
| **4: CSEK SEQ ID NO:7** | YFGKLASKLSVIRNLNDQVLFIDQGNRPLFEDMTDSDSRDNAPRTIFIIS MYADSQPRGMAVTISVKSEKISTLSSENKIISFKEMNPPDNIKDTKSDIIF FQRSVPGHDNKMQFESSSYEGYFLASEKERDLFKLILKKEDELGDRSI MFTVQNED |
| **5B-1: CSEK-G3Y SEQ ID NO:9** | YFYKLASKLSVIRNLNDQVLFIDQGNRPLFEDMTDSDSRDNAPRTIFIIS MYADSQPRGMAVTISVKSEKISTLSSENKIISFKEMNPPDNIKDTKSDIIF FQRSVPGHDNKMQFESSSYEGYFLASEKERDLFKLILKKEDELGDRSI MFTVQNED |
| **5B-2: CSEK-G3L SEQ ID NO:11** | YFLKLASKLSVIRNLNDQVLFIDQGNRPLFEDMTDSDSRDNAPRTIFIIS MYADSQPRGMAVTISVKSEKISTLSSENKIISFKEMNPPDNIKDTKSDIIF FQRSVPGHDNKMQFESSSYEGYFLASEKERDLFKLILKKEDELGDRSI MFTVQNED |
| **5B-3: CSEK-A6W (E6W) SEQ ID NO:13** | YFGKLWSKLSVIRNLNDQVLFIDQGNRPLFEDMTDSDSRDNAPRTIFIIS MYADSQPRGMAVTISVKSEKISTLSSENKIISFKEMNPPDNIKDTKSDIIF FQRSVPGHDNKMQFESSSYEGYFLASEKERDLFKLILKKEDELGDRSI MFTVQNED |
| **5B-4: CSEK-T34P SEQ ID NO:15** | YFGKLASKLSVIRNLNDQVLFIDQGNRPLFEDMPDSDSRDNAPRTIFIIS MYADSQPRGMAVTISVKSEKISTLSSENKIISFKEMNPPDNIKDTKSDIIF FQRSVPGHDNKMQFESSSYEGYFLASEKERDLFKLILKKEDELGDRSI MFTVQNED |
| **5B-5: CSEK-C38M SEQ ID NO:17** | YFGKLASKLSVIRNLNDQVLFIDQGNRPLFEDMTDSDMRDNAPRTIFIIS MYADSQPRGMAVTISVKSEKISTLSSENKIISFKEMNPPDNIKDTKSDIIF FQRSVPGHDNKMQFESSSYEGYFLASEKERDLFKLILKKEDELGDRSI MFTVQNED |
| 5B-6: **CSEK-M51Y** | YFGKLASKLSVIRNLNDQVLFIDQGNRPLFEDMTDSDSRDNAPRTIFIIS YYADSQPRGMAVTISVKSEKISTLSSENKIISFKEMNPPDNIKDTKSDIIFF |
| **SEQ ID NO:19** | QRSVPGHDNKMQFESSSYEGYFLASEKERDLFKLILKKEDELGDRSIM FTVQNED |
| **5B-7: CSEK-S72Y SEQ ID NO:21** | YFGKLASKLSVIRNLNDQVLFIDQGNRPLFEDMTDSDSRDNAPRTIFIIS MYADSQPRGMAVTISVKSEKIYTLSSENKIISFKEMNPPDNIKDTKSDIIF FQRSVPGHDNKMQFESSSYEGYFLASEKERDLFKLILKKEDELGDRSI MFTVQNED |
| **5B-8: CSEK-S72F SEQ ID NO:23** | YFGKLASKLSVIRNLNDQVLFIDQGNRPLFEDMTDSDSRDNAPRTIFIIS MYADSQPRGMAVTISVKSEKIFTLSSENKIISFKEMNPPDNIKDTKSDIIF FQRSVPGHDNKMQFESSSYEGYFLASEKERDLFKLILKKEDELGDRSI MFTVQNED |
| **5B-9: CSEK-S72M SEQ ID NO:25** | YFGKLASKLSVIRNLNDQVLFIDQGNRPLFEDMTDSDSRDNAPRTIFIIS MYADSQPRGMAVTISVKSEKIMTLSSENKIISFKEMNPPDNIKDTKSDIIF FQRSVPGHDNKMQFESSSYEGYFLASEKERDLFKLILKKEDELGDRSI MFTVQNED |
| **5B-10: CSEK-S72L SEQ ID NO:27** | YFGKLASKLSVIRNLNDQVLFIDQGNRPLFEDMTDSDSRDNAPRTIFIIS MYADSQPRGMAVTISVKSEKILTLSSENKIISFKEMNPPDNIKDTKSDIIF FQRSVPGHDNKMQFESSSYEGYFLASEKERDLFKLILKKEDELGDRSI MFTVQNED |
| **5B-11: CSEK-S72W SEQ ID NO:29** | YFGKLASKLSVIRNLNDQVLFIDQGNRPLFEDMTDSDSRDNAPRTIFIIS MYADSQPRGMAVTISVKSEKIWTLSSENKIISFKEMNPPDNIKDTKSDIIF FQRSVPGHDNKMQFESSSYEGYFLASEKERDLFKLILKKEDELGDRSI MFTVQNED |
| **5B-12: CSEK-K112W SEQ ID NO:31** | YFGKLASKLSVIRNLNDQVLFIDQGNRPLFEDMTDSDSRDNAPRTIFIIS MYADSQPRGMAVTISVKSEKISTLSSENKIISFKEMNPPDNIKDTKSDIIF FQRSVPGHDNWMQFESSSYEGYFLASEKERDLFKLILKKEDELGDRSI MFTVQNED |
| **5B-13: CSEK-S119V SEQ ID NO:33** | YFGKLASKLSVIRNLNDQVLFIDQGNRPLFEDMTDSDSRDNAPRTIFIIS MYADSQPRGMAVTISVKSEKISTLSSENKIISFKEMNPPDNIKDTKSDIIF FQRSVPGHDNKMQFESSVYEGYFLASEKERDLFKLILKKEDELGDRSI MFTVQNED |
| **5B-14: CSEK-G145N SEQ ID NO:35** | YFGKLASKLSVIRNLNDQVLFIDQGNRPLFEDMTDSDSRDNAPRTIFIIS MYADSQPRGMAVTISVKSEKISTLSSENKIISFKEMNPPDNIKDTKSDIIF FQRSVPGHDNKMQFESSSYEGYFLASEKERDLFKLILKKEDELNDRSI MFTVQNED |
| **5B-15: CSEK-S7/50C SEQ ID NO:37** | YFGKLACKLSVIRNLNDQVLFIDQGNRPLFEDMTDSDSRDNAPRTIFIIC MYADSQPRGMAVTISVKSEKISTLSSENKIISFKEMNPPDNIKDTKSDIIF FQRSVPGHDNKMQFESSSYEGYFLASEKERDLFKLILKKEDELGDRSI MFTVQNED |
| **12-1 CS-G3Y SEQ ID NO:45** | YFYKLESKLSVIRNLNDQVLFIDQGNRPLFEDMTDSDSRDNAPRTIFIIS MYKDSQPRGMAVTISVKSEKISTLSSENKIISFKEMNPPDNIKDTKSDIIF FQRSVPGHDNKMQFESSSYEGYFLASEKERDLFKLILKKEDELGDRSI MFTVQNED |
| **12-2 CS-G3L SEQ ID NO:47** | YFLKLESKLSVIRNLNDQVLFIDQGNRPLFEDMTDSDSRDNAPRTIFIIS MYKDSQPRGMAVTISVKSEKISTLSSENKIISFKEMNPPDNIKDTKSDIIF FQRSVPGHDNKMQFESSSYEGYFLASEKERDLFKLILKKEDELGDRSI MFTVQNED |
| **12-3 CS-C38M SEQ ID NO:49** | YFGKLESKLSVIRNLNDQVLFIDQGNRPLFEDMTDSDMRDNAPRTIFIIS MYKDSQPRGMAVTISVKSEKISTLSSENKIISFKEMNPPDNIKDTKSDIIF FQRSVPGHDNKMQFESSSYEGYFLASEKERDLFKLILKKEDELGDRSI MFTVQNED |
| **12-4 CS-S72Y SEQ ID NO:51** | YFGKLESKLSVIRNLNDQVLFIDQGNRPLFEDMTDSDSRDNAPRTIFIIS MYKDSQPRGMAVTISVKSEKIYTLSSENKIISFKEMNPPDNIKDTKSDIIF FQRSVPGHDNKMQFESSSYEGYFLASEKERDLFKLILKKEDELGDRSI MFTVQNED |
| **12-5 CS-S72F SEQ ID NO:53** | YFGKLESKLSVIRNLNDQVLFIDQGNRPLFEDMTDSDSRDNAPRTIFIIS MYKDSQPRGMAVTISVKSEKIFTLSSENKIISFKEMNPPDNIKDTKSDIIF FQRSVPGHDNKMQFESSSYEGYFLASEKERDLFKLILKKEDELGDRSI MFTVQNED |
| **12-6 CS-S72M SEQ ID NO:55** | YFGKLESKLSVIRNLNDQVLFIDQGNRPLFEDMTDSDSRDNAPRTIFIIS MYKDSQPRGMAVTISVKSEKIMTLSSENKIISFKEMNPPDNIKDTKSDIIF FQRSVPGHDNKMQFESSSYEGYFLASEKERDLFKLILKKEDELGDRSI MFTVQNED |
| **12-7 G3Y SEQ ID NO:57** | YFYKLESKLSVIRNLNDQVLFIDQGNRPLFEDMTDSDCRDNAPRTIFIIS MYKDSQPRGMAVTISVKCEKISTLSCENKIISFKEMNPPDNIKDTKSDIIF FQRSVPGHDNKMQFESSSYEGYFLACEKERDLFKLILKKEDELGDRSI MFTVQNED |
| **12-8 G3L SEQ ID NO:59** | YFLKLESKLSVIRNLNDQVLFIDQGNRPLFEDMTDSDCRDNAPRTIFIIS MYKDSQPRGMAVTISVKCEKISTLSCENKIISFKEMNPPDNIKDTKSDIIF FQRSVPGHDNKMQFESSSYEGYFLACEKERDLFKLILKKEDELGDRSI MFTVQNED |
| **12-9 C38M SEQ ID NO:61** | YFGKLESKLSVIRNLNDQVLFIDQGNRPLFEDMTDSDMRDNAPRTIFIIS MYKDSQPRGMAVTISVKCEKISTLSCENKIISFKEMNPPDNIKDTKSDIIF FQRSVPGHDNKMQFESSSYEGYFLACEKERDLFKLILKKEDELGDRSI MFTVQNED |
| **12-10 S72Y SEQ ID NO:63** | YFGKLESKLSVIRNLNDQVLFIDQGNRPLFEDMTDSDCRDNAPRTIFIIS MYKDSQPRGMAVTISVKCEKIYTLSCENKIISFKEMNPPDNIKDTKSDIIF FQRSVPGHDNKMQFESSSYEGYFLACEKERDLFKLILKKEDELGDRSI MFTVQNED |
| **12-11 CS-S72F SEQ ID NO:65** | YFGKLESKLSVIRNLNDQVLFIDQGNRPLFEDMTDSDCRDNAPRTIFIIS MYKDSQPRGMAVTISVKCEKIETLSCENKIISFKEMNPPDNIKDTKSDIIF FQRSVPGHDNKMQFESSSYEGYFLACEKERDLFKLILKKEDELGDRSI MFTVQNED |
| **12-12 S72M SEQ ID NO:67** | YFGKLESKLSVIRNLNDQVLFIDQGNRPLFEDMTDSDCRDNAPRTIFIIS MYKDSQPRGMAVTISVKCEKIMTLSCENKIISFKEMNPPDNIKDTKSDIIF FQRSVPGHDNKMQFESSSYEGYFLACEKERDLFKLILKKEDELGDRSI MFTVQNED |

**[Table 4]**

| **Example #** | **DNA sequence encoding hIL18 variant (without propeptide)** |
|---|---|
| **3: CS SEQ ID NO:6** | tacttcggtaaactggaatctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacaaagactctcagccgcgtggtatggctgttacca tctctgttaaatctgaaaaaatctctaccctgtcttctgaaaacaaaatcatctctttcaaagaa atgaacccgccggacaacatcaaagacaccaaatctgacatcatcttcttccagcgttctgtt ccgggtcacgacaacaaaatgcagttcgaatcttcttcttacgaaggttacttcctggcttctg aaaaagaacgtgacctgttcaaactgatcctgaaaaaagaagacgaactgggtgaccgttc tatcatgttcaccgttcagaacgaagactaagtcgac |
| **4: CSEK SEQ ID NO:8** | tacttcggtaaactggcttctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacgctgactctcagccgcgtggtatggctgttacca tctctgttaaatctgaaaaaatctctaccctgtcttctgaaaacaaaatcatctctttcaaagaaa tgaacccgccggacaacatcaaagacaccaaatctgacatcatcttcttccagcgttctgttc cgggtcacgacaacaaaatgcagttcgaatcttcttcttacgaaggttacttcctggcttctga aaaagaacgtgacctgttcaaactgatcctgaaaaaagaagacgaactgggtgaccgttct atcatgttcaccgttcagaacgaagactaagtcgac |
| **5B-1: CSEK-G3Y SEQ ID NO:10** | tacttctacaaactggcttctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacgctgactctcagccgcgtggtatggctgttacca tctctgttaaatctgaaaaaatctctaccctgtcttctgaaaacaaaatcatctctttcaaagaaa tgaacccgccggacaacatcaaagacaccaaatctgacatcatcttcttccagcgttctgttc cgggtcacgacaacaaaatgcagttcgaatcttcttcttacgaaggttacttcctggcttctga aaaagaacgtgacctgttcaaactgatcctgaaaaaagaagacgaactgggtgaccgttct atcatgttcaccgttcagaacgaagactaagtcgac |
| **B-2: CSEK-G3L SEQ ID NO:12** | tacttcctgaaactggcttctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacgctgactctcagccgcgtggtatggctgttacca tctctgttaaatctgaaaaaatctctaccctgtcttctgaaaacaaaatcatctctttcaaagaaa tgaacccgccggacaacatcaaagacaccaaatctgacatcatcttcttccagcgttctgttc cgggtcacgacaacaaaatgcagttcgaatcttcttcttacgaaggttacttcctggcttctga aaaagaacgtgacctgttcaaactgatcctgaaaaaagaagacgaactgggtgaccgttct atcatgttcaccgttcagaacgaagactaagtcgac |
| **B-3: CSEK-A6W SEQ ID NO:14** | tacttcggtaaactgtggtctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacgctgactctcagccgcgtggtatggctgttacca tctctgttaaatctgaaaaaatctctaccctgtcttctgaaaacaaaatcatctctttcaaagaaa tgaacccgccggacaacatcaaagacaccaaatctgacatcatcttcttccagcgttctgttc cgggtcacgacaacaaaatgcagttcgaatcttcttcttacgaaggttacttcctggcttctga aaaagaacgtgacctgttcaaactgatcctgaaaaaagaagacgaactgggtgaccgttct atcatgttcaccgttcagaacgaagactaagtcgac |
| **B-4: CSEK-T34P SEQ ID NO:16** | tacttcggtaaactggcttctaaactgtctgttatccgtaacctgaacgaccaggttctgttcat cgaccagggtaaccgtccgctgttcgaagacatgccggactctgactctcgtgacaacgct ccgcgtaccatcttcatcatctctatgtacgctgactctcagccgcgtggtatggctgttacca tctctgttaaatctgaaaaaatctctaccctgtcttctgaaaacaaaatcatctctttcaaagaaa tgaacccgccggacaacatcaaagacaccaaatctgacatcatcttcttccagcgttctgttc cgggtcacgacaacaaaatgcagttcgaatcttcttcttacgaaggttacttcctggcttctga aaaagaacgtgacctgttcaaactgatcctgaaaaaagaagacgaactgggtgaccgttct atcatgttcaccgttcagaacgaagactaagtcgac |
| **B-5: CSEK-C38M SEQ ID NO:18** | tacttcggtaaactggcttctaaactgtctgttatccgtaacctgaacgaccaggttctgttcat cgaccagggtaaccgtccgctgttcgaagacatgaccgactctgacatgcgtgacaacgc tccgcgtaccatcttcatcatctctatgtacgctgactctcagccgcgtggtatggctgttacc atctctgttaaatctgaaaaaatctctaccctgtcttctgaaaacaaaatcatctctttcaaagaa atgaacccgccggacaacatcaaagacaccaaatctgacatcatcttcttccagcgttctgtt ccgggtcacgacaacaaaatgcagttcgaatcttcttcttacgaaggttacttcctggcttctg aaaaagaacgtgacctgttcaaactgatcctgaaaaaagaagacgaactgggtgaccgttc tatcatgttcaccgttcagaacgaagactaagtcgac |
| **B-6: CSEK-M51Y SEQ ID NO:20** | tacttcggtaaactggcttctaaactgtctgttatccgtaacctgaacgaccaggttctgttcat cgaccagggtaaccgtccgctgttcgaagacatgaccgactctgactctcgtgacaacgct ccgcgtaccatcttcatcatctcttactacgctgactctcagccgcgtggtatggctgttacca tctctgttaaatctgaaaaaatctctaccctgtcttctgaaaacaaaatcatctctttcaaagaaa tgaacccgccggacaacatcaaagacaccaaatctgacatcatcttcttccagcgttctgttc cgggtcacgacaacaaaatgcagttcgaatcttcttcttacgaaggttacttcctggcttctga aaaagaacgtgacctgttcaaactgatcctgaaaaaagaagacgaactgggtgaccgttct atcatgttcaccgttcagaacgaagactaagtcgac |
| **B-7: CSEK-S72Y SEQ ID NO:22** | tacttcggtaaactggcttctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacgctgactctcagccgcgtggtatggctgttacca tctctgttaaatctgaaaaaatctacaccctgtcttctgaaaacaaaatcatctctttcaaagaa atgaacccgccggacaacatcaaagacaccaaatctgacatcatcttcttccagcgttctgtt ccgggtcacgacaacaaaatgcagttcgaatcttcttcttacgaaggttacttcctggcttctg aaaaagaacgtgacctgttcaaactgatcctgaaaaaagaagacgaactgggtgaccgttc tatcatgttcaccgttcagaacgaagactaagtcgac |
| **B-8: CSEK-S72F SEQ ID NO:24** | tacttcggtaaactggcttctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacgctgactctcagccgcgtggtatggctgttacca tctctgttaaatctgaaaaaatcttcaccctgtcttctgaaaacaaaatcatctctttcaaagaa atgaacccgccggacaacatcaaagacaccaaatctgacatcatcttcttccagcgttctgtt ccgggtcacgacaacaaaatgcagttcgaatcttcttcttacgaaggttacttcctggcttctg aaaaagaacgtgacctgttcaaactgatcctgaaaaaagaagacgaactgggtgaccgttc tatcatgttcaccgttcagaacgaagactaagtcgac |
| | |
| **B-9: CSEK-S72M SEQ ID NO:26** | tacttcggtaaactggcttctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacgctgactctcagccgcgtggtatggctgttacca tctctgttaaatctgaaaaaatcatgaccctgtcttctgaaaacaaaatcatctctttcaaagaa atgaacccgccggacaacatcaaagacaccaaatctgacatcatcttcttccagcgttctgtt ccgggtcacgacaacaaaatgcagttcgaatcttcttcttacgaaggttacttcctggcttctg aaaaagaacgtgacctgttcaaactgatcctgaaaaaagaagacgaactgggtgaccgttc tatcatgttcaccgttcagaacgaagactaagtcgac |
| **B-10: CSEK-S72W SEQ ID NO:28** | tacttcggtaaactggcttctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacgctgactctcagccgcgtggtatggctgttacca tctctgttaaatctgaaaaaatcctgaccctgtcttctgaaaacaaaatcatctctttcaaagaa atgaacccgccggacaacatcaaagacaccaaatctgacatcatcttcttccagcgttctgtt ccgggtcacgacaacaaaatgcagttcgaatcttcttcttacgaaggttacttcctggcttctg aaaaagaacgtgacctgttcaaactgatcctgaaaaaagaagacgaactgggtgaccgttc tatcatgttcaccgttcagaacgaagactaagtcgac |
| **B-11: CSEK-S72W SEQ ID NO:30** | tacttcggtaaactggcttctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacgctgactctcagccgcgtggtatggctgttacca tctctgttaaatctgaaaaaatctggaccctgtcttctgaaaacaaaatcatctctttcaaagaa atgaacccgccggacaacatcaaagacaccaaatctgacatcatcttcttccagcgttctgtt ccgggtcacgacaacaaaatgcagttcgaatcttcttcttacgaaggttacttcctggcttctg aaaaagaacgtgacctgttcaaactgatcctgaaaaaagaagacgaactgggtgaccgttc tatcatgttcaccgttcagaacgaagactaagtcgac |
| **B-12: CSEK-K112W SEQ ID NO:32** | tacttcggtaaactggcttctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacgctgactctcagccgcgtggtatggctgttacca tctctgttaaatctgaaaaaatctctaccctgtcttctgaaaacaaaatcatctctttcaaagaaa tgaacccgccggacaacatcaaagacaccaaatctgacatcatcttcttccagcgttctgttc cgggtcacgacaactggatgcagttcgaatcttcttcttacgaaggttacttcctggcttctga aaaagaacgtgacctgttcaaactgatcctgaaaaaagaagacgaactgggtgaccgttct atcatgttcaccgttcagaacgaagactaagtcgac |
| **B-13: CSEK-S119V SEQ ID NO:34** | tacttcggtaaactggcttctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacgctgactctcagccgcgtggtatggctgttacca tctctgttaaatctgaaaaaatctctaccctgtcttctgaaaacaaaatcatctctttcaaagaaa tgaacccgccggacaacatcaaagacaccaaatctgacatcatcttcttccagcgttctgttc cgggtcacgacaacaaaatgcagttcgaatcttctgtttacgaaggttacttcctggcttctga aaaagaacgtgacctgttcaaactgatcctgaaaaaagaagacgaactgggtgaccgttct atcatgttcaccgttcagaacgaagactaagtcgac |
| **B-14: CSEK-G145N SEQ ID NO:36** | tacttcggtaaactggcttctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacgctgactctcagccgcgtggtatggctgttacca tctctgttaaatctgaaaaaatctctaccctgtcttctgaaaacaaaatcatctctttcaaagaaa tgaacccgccggacaacatcaaagacaccaaatctgacatcatcttcttccagcgttctgttc cgggtcacgacaacaaaatgcagttcgaatcttcttcttacgaaggttacttcctggcttctga aaaagaacgtgacctgttcaaactgatcctgaaaaaagaagacgaactgaacgaccgttct atcatgttcaccgttcagaacgaagactaagtcgac |
| **B-15: CSEK-S7/50C SEQ ID NO:38** | tacttcggtaaactggcttgcaaactgtctgttatccgtaacctgaacgaccaggttctgttcat cgaccagggtaaccgtccgctgttcgaagacatgaccgactctgactctcgtgacaacgct ccgcgtaccatcttcatcatctgcatgtacgctgactctcagccgcgtggtatggctgttacc atctctgttaaatctgaaaaaatctctaccctgtcttctgaaaacaaaatcatctctttcaaagaa atgaacccgccggacaacatcaaagacaccaaatctgacatcatcttcttccagcgttctgtt ccgggtcacgacaacaaaatgcagttcgaatcttcttcttacgaaggttacttcctggcttctg |
| | aaaaagaacgtgacctgttcaaactgatcctgaaaaaagaagacgaactgggtgaccgttc tatcatgttcaccgttcagaacgaagactaagtcgac |
| **12-1 CS-G3Y SEQ ID NO:46** | tacttctacaaactggaatctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacaaagactctcagccgcgtggtatggctgttacca tctctgttaaatctgaaaaaatctctaccctgtcttctgaaaacaaaatcatctctttcaaagaaa tgaacccgccggacaacatcaaagacaccaaatctgacatcatcttcttccagcgttctgttc cgggtcacgacaacaaaatgcagttcgaatcttcttcttacgaaggttacttcctggcttctga aaaagaacgtgacctgttcaaactgatcctgaaaaaagaagacgaactgggtgaccgttct atcatgttcaccgttcagaacgaagactaagtcgac |
| **12-2 CS-G3L SEQ ID NO:48** | tacttcctgaaactggaatctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacaaagactctcagccgcgtggtatggctgttacca tctctgttaaatctgaaaaaatctctaccctgtcttctgaaaacaaaatcatctctttcaaagaaa tgaacccgccggacaacatcaaagacaccaaatctgacatcatcttcttccagcgttctgttc cgggtcacgacaacaaaatgcagttcgaatcttcttcttacgaaggttacttcctggcttctga aaaagaacgtgacctgttcaaactgatcctgaaaaaagaagacgaactgggtgaccgttct atcatgttcaccgttcagaacgaagactaagtcgac |
| **12-3 CS-C38M SEQ ID NO:50** | tacttcggtaaactggaatctaaactgtctgttatccgtaacctgaacgaccaggttctgttcat cgaccagggtaaccgtccgctgttcgaagacatgaccgactctgacatgcgtgacaacgc tccgcgtaccatcttcatcatctctatgtacaaagactctcagccgcgtggtatggctgttacc atctctgttaaatctgaaaaaatctctaccctgtcttctgaaaacaaaatcatctctttcaaagaa atgaacccgccggacaacatcaaagacaccaaatctgacatcatcttcttccagcgttctgtt ccgggtcacgacaacaaaatgcagttcgaatcttcttcttacgaaggttacttcctggcttctg aaaaagaacgtgacctgttcaaactgatcctgaaaaaagaagacgaactgggtgaccgttc tatcatgttcaccgttcagaacgaagactaagtcgac |
| **12-4 CS-S72Y SEQ ID NO:52** | tacttcggtaaactggaatctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacaaagactctcagccgcgtggtatggctgttacca tctctgttaaatctgaaaaaatctacaccctgtcttctgaaaacaaaatcatctctttcaaagaa atgaacccgccggacaacatcaaagacaccaaatctgacatcatcttcttccagcgttctgtt ccgggtcacgacaacaaaatgcagttcgaatcttcttcttacgaaggttacttcctggcttctg aaaaagaacgtgacctgttcaaactgatcctgaaaaaagaagacgaactgggtgaccgttc tatcatgttcaccgttcagaacgaagactaagtcgac |
| **12-5 CS-S72F SEQ ID NO:54** | tacttcggtaaactggaatctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacaaagactctcagccgcgtggtatggctgttacca tctctgttaaatctgaaaaaatcttcaccctgtcttctgaaaacaaaatcatctctttcaaagaa atgaacccgccggacaacatcaaagacaccaaatctgacatcatcttcttccagcgttctgtt ccgggtcacgacaacaaaatgcagttcgaatcttcttcttacgaaggttacttcctggcttctg aaaaagaacgtgacctgttcaaactgatcctgaaaaaagaagacgaactgggtgaccgttc tatcatgttcaccgttcagaacgaagactaagtcgac |
| **12-6 CS-S72M SEQ ID NO:56** | tacttcggtaaactggaatctaaactgtctgttatccgtaacctgaacgaccaggttctgttcat cgaccagggtaaccgtccgctgttcgaagacatgaccgactctgactctcgtgacaacgct ccgcgtaccatcttcatcatctctatgtacaaagactctcagccgcgtggtatggctgttacca tctctgttaaatctgaaaaaatcatgaccctgtcttctgaaaacaaaatcatctctttcaaagaa atgaacccgccggacaacatcaaagacaccaaatctgacatcatcttcttccagcgttctgtt ccgggtcacgacaacaaaatgcagttcgaatcttcttcttacgaaggttacttcctggcttctg aaaaagaacgtgacctgttcaaactgatcctgaaaaaagaagacgaactgggtgaccgttc tatcatgttcaccgttcagaacgaagactaagtcgac |
| **12-7 G3Y SEQ ID NO:58** | tacttctacaaactggaatctaaactgtctgttatccgtaacctgaacgaccaggttctgttcat cgaccagggtaaccgtccgctgttcgaagacatgaccgactctgactgccgtgacaacgct ccgcgtaccatcttcatcatctctatgtacaaagactctcagccgcgtggtatggctgttacca tctctgttaaatgcgaaaaaatctctaccctgtcttgcgaaaacaaaatcatctctttcaaagaa atgaacccgccggacaacatcaaagacaccaaatctgacatcatcttcttccagcgttctgtt |
| | ccgggtcacgacaacaaaatgcagttcgaatcttcttcttacgaaggttacttcctggcttgcg aaaaagaacgtgacctgttcaaactgatcctgaaaaaagaagacgaactgggtgaccgttc tatcatgttcaccgttcagaacgaagactaagtcgac |
| **12-8 G3L SEQ ID NO:60** | tacttcctgaaactggaatctaaactgtctgttatccgtaacctgaacgaccaggttctgttcat cgaccagggtaaccgtccgctgttcgaagacatgaccgactctgactgccgtgacaacgct ccgcgtaccatcttcatcatctctatgtacaaagactctcagccgcgtggtatggctgttacca tctctgttaaatgcgaaaaaatctctaccctgtcttgcgaaaacaaaatcatctctttcaaagaa atgaacccgccggacaacatcaaagacaccaaatctgacatcatcttcttccagcgttctgtt ccgggtcacgacaacaaaatgcagttcgaatcttcttcttacgaaggttacttcctggcttgcg aaaaagaacgtgacctgttcaaactgatcctgaaaaaagaagacgaactgggtgaccgttc tatcatgttcaccgttcagaacgaagactaagtcgac |
| **12-9 C38M SEQ ID NO:62** | tacttcggtaaactggaatctaaactgtctgttatccgtaacctgaacgaccaggttctgttcat cgaccagggtaaccgtccgctgttcgaagacatgaccgactctgacatgcgtgacaacgc tccgcgtaccatcttcatcatctctatgtacaaagactctcagccgcgtggtatggctgttacc atctctgttaaatgcgaaaaaatctctaccctgtcttgcgaaaacaaaatcatctctttcaaaga aatgaacccgccggacaacatcaaagacaccaaatctgacatcatcttcttccagcgttctg ttccgggtcacgacaacaaaatgcagttcgaatcttcttcttacgaaggttacttcctggcttg cgaaaaagaacgtgacctgttcaaactgatcctgaaaaaagaagacgaactgggtgaccg ttctatcatgttcaccgttcagaacgaagactaagtcgac |
| **12-10 S72Y SEQ ID NO:64** | tacttcggtaaactggaatctaaactgtctgttatccgtaacctgaacgaccaggttctgttcat cgaccagggtaaccgtccgctgttcgaagacatgaccgactctgactgccgtgacaacgct ccgcgtaccatcttcatcatctctatgtacaaagactctcagccgcgtggtatggctgttacca tctctgttaaatgcgaaaaaatctacaccctgtcttgcgaaaacaaaatcatctctttcaaaga aatgaacccgccggacaacatcaaagacaccaaatctgacatcatcttcttccagcgttctg ttccgggtcacgacaacaaaatgcagttcgaatcttcttcttacgaaggttacttcctggcttg cgaaaaagaacgtgacctgttcaaactgatcctgaaaaaagaagacgaactgggtgaccg ttctatcatgttcaccgttcagaacgaagactaagtcgac |
| **12-11 S72F SEQ ID NO:66** | tacttcggtaaactggaatctaaactgtctgttatccgtaacctgaacgaccaggttctgttcat cgaccagggtaaccgtccgctgttcgaagacatgaccgactctgactgccgtgacaacgct ccgcgtaccatcttcatcatctctatgtacaaagactctcagccgcgtggtatggctgttacca tctctgttaaatgcgaaaaaatcttcaccctgtcttgcgaaaacaaaatcatctctttcaaaga aatgaacccgccggacaacatcaaagacaccaaatctgacatcatcttcttccagcgttctg ttccgggtcacgacaacaaaatgcagttcgaatcttcttcttacgaaggttacttcctggcttg cgaaaaagaacgtgacctgttcaaactgatcctgaaaaaagaagacgaactgggtgaccg ttctatcatgttcaccgttcagaacgaagactaagtcgac |
| **12-12 S72M SEQ ID NO:68** | tacttcggtaaactggaatctaaactgtctgttatccgtaacctgaacgaccaggttctgttcat cgaccagggtaaccgtccgctgttcgaagacatgaccgactctgactgccgtgacaacgct ccgcgtaccatcttcatcatctctatgtacaaagactctcagccgcgtggtatggctgttacca tctctgttaaatgcgaaaaaatcatgaccctgtcttgcgaaaacaaaatcatctctttcaaaga aatgaacccgccggacaacatcaaagacaccaaatctgacatcatcttcttccagcgttctg ttccgggtcacgacaacaaaatgcagttcgaatcttcttcttacgaaggttacttcctggcttg cgaaaaagaacgtgacctgttcaaactgatcctgaaaaaagaagacgaactgggtgaccg ttctatcatgttcaccgttcagaacgaagactaagtcgac |

### Industrial Applicability

The human IL-18 variant of the present invention is highly stable and highly active, thus it is useful for the treatment of cancers, viral diseases, immune diseases, and the like.

All the publications, patents, and patent applications cited herein are incorporated herein by reference in its entirety.

### Free Text of Sequence Listing

SEQ ID NO:4 : NdeI-hProIL18-SalI
SEQ ID NO:5 : hIL-18-CS
SEQ ID NO:6 : NdeI-hProIL18-CS-SalI
SEQ ID NO:7 : hIL-18-CSEK
SEQ ID NO:8 : NdeI-hProIL18-CSEK-SalI
SEQ ID NO:9 : hIL-18-CSEK-G3Y
SEQ ID NO:10 : NdeI-hProIL18-CSEK-G3Y-SalI
SEQ ID NO:11 : hIL-18-CSEK-G3L
SEQ ID NO:12 : NdeI-hProIL18-CSEK-G3L-SalI
SEQ ID NO:13 : hIL-18-CSEK-A6W
SEQ ID NO:14 : NdeI-hProIL18-CSEK-A6W-SalI
SEQ ID NO:15 : hIL-18-CSEK-T34P
SEQ ID NO:16 : NdeI-hProIL18CSEK-T34P-SalI
SEQ ID NO:17 : hIL-18-CSEK-C38M
SEQ ID NO:18 : NdeI-hProIL18CSEK-C38M-SalI
SEQ ID NO:19 : hIL-18-CSEK-M51Y
SEQ ID NO:20 : NdeI-hProIL18CSEK-M51Y-SalI
SEQ ID NO:21 : hIL-18-CSEK-S72Y
SEQ ID NO:22 : NdeI-hProIL18CSEK-S72Y-SalI
SEQ ID NO:23 : hIL-18-CSEK-S72F
SEQ ID NO:24 : NdeI-hProIL18CSEK-S72F-SalI
SEQ ID NO:25 : hIL-18-CSEK-S72M
SEQ ID NO:26 : NdeI-hProIL18CSEK-S72M-SalI
SEQ ID NO:27 : hIL-18-CSEK-S72L
SEQ ID NO:28 : NdeI-hProIL18CSEK-S72L-SalI
SEQ ID NO:29 : hIL-18-CSEK-S72W
SEQ ID NO:30 : NdeI-hProIL18CSEK-S72W-SalI
SEQ ID NO:31 : hIL-18-CSEK-K112W
SEQ ID NO:32 : NdeI-hProIL18CSEK-K112W-SalI
SEQ ID NO:33 : hIL-18-CSEK-K119V
SEQ ID NO:34 : NdeI-hProIL18CSEK-K119V-SalI
SEQ ID NO:35 : hIL-18-CSEK-G145N
SEQ ID NO:36 : NdeI-hProIL18CSEK-G145N-SalI
SEQ ID NO:37 : hIL-18-CSEK-S7/50C
SEQ ID NO:38 : NdeI-hProIL18CSEK-S7/50C-SalI
SEQ ID NO:40 : hCasp4-D315E
SEQ ID NO:41 : hCasp4-D315E DNA
SEQ ID NO:42 : NdeI-hCasp4-D315E-SalI
SEQ ID NO:45 : hIL-18-CS-G3Y
SEQ ID NO:46 : NdeI-hProIL18CS-G3Y-SalI
SEQ ID NO:47 : hIL-18-CS-G3L
SEQ ID NO:48 : NdeI-hProIL18CS-G3L-SalI
SEQ ID NO:49 : hIL-18-CS-C38M
SEQ ID NO:50 : NdeI-hProIL18CS-C38M-SalI
SEQ ID NO:51 : hIL-18-CS-S72Y
SEQ ID NO:52 : NdeI-hProIL18CS-S72Y-SalI
SEQ ID NO:53 : hIL-18-CS-S72F
SEQ ID NO:54 : NdeI-hProIL18CS-S72F-SalI
SEQ ID NO:55 : hIL-18-CS-S72M
SEQ ID NO:56 : NdeI-hProIL18CS-S72M-SalI
SEQ ID NO:57 : hIL-18-G3Y
SEQ ID NO:58 : NdeI-hProIL18-G3Y-SalI
SEQ ID NO:59 : hIL-18-G3L
SEQ ID NO:60 : NdeI-hProIL18-G3L-SalI
SEQ ID NO:61 : hIL-18-C38M
SEQ ID NO:62 : NdeI-hProIL18-C38M-SalI
SEQ ID NO:63 : hIL-18-S72Y
SEQ ID NO:64 : NdeI-hProIL18-S72Y-SalI
SEQ ID NO:65 : hIL-18-S72F
SEQ ID NO:66 : NdeI-hProIL18-S72F-SalI
SEQ ID NO:67 : hIL-18-S72M
SEQ ID NO:68 : NdeI-hProIL18-S72M-SalI

## Claims

1. A human IL-18 variant comprising an amino acid sequence having substitutions of cysteine at positions 38, 68, 76, and 127 with serine and substitutions of glutamic acid at position 6 and lysine at position 53 with alanine relative to an amino acid sequence of wild-type human IL-18,
wherein the variant has at least one additional mutation introduced therein, and has a higher activity than the wild-type human IL-18.

2. The human IL-18 variant according to claim 1, wherein the additional mutation is selected from mutations at positions 3, 34, 38, 51, 72, 112, 119, and 145 and an introduction of a disulfide bond between positions 7 and 50 in the amino acid sequence of the wild-type human IL-18.

3. The human IL-18 variant according to claim 1, wherein the additional mutation is selected from G3Y, G3L, A6W, T34P, C38M, M51Y, S72Y, S72F, S72M, S72L, S72W, K112W, K119V, G145N, and S7/50C.

4. The human IL-18 variant according to any one of claims 1 to 3, wherein the activity is an ability of enhancing interferon y production induction.

5. A human IL-18 variant comprising an amino acid sequence having substitutions of cysteine at positions 38, 68, 76, and 127 with serine and substitutions of glutamic acid at position 6 and lysine at position 53 with alanine relative to an amino acid sequence of wild-type human IL-18,
wherein the variant has at least one additional mutation introduced therein, and the additional mutation is any one selected from G3Y, G3L, A6W, T34P, C38M, M51Y, S72Y, S72F, S72M, S72L, S72W, K112W, K119V, G145N, and S7/50C.

6. The human IL-18 variant according to any one of claims 1 to 5, wherein the additional mutation is any one selected from G3Y, G3L, C38M, S72Y, S72F, S72M, S72L, and S72W.

7. The human IL-18 variant according to any one of claims 1 to 6, wherein the amino acid sequence of the wild-type human IL-18 includes an amino acid sequence represented by SEQ ID NO: 3.

8. A pharmaceutical composition comprising the human IL-18 variant according to any one of claims 1 to 7.

9. The pharmaceutical composition according to claim 8, for treating a viral disease, a cancer, or an immune disease which are sensitive to IFNy.

10. The pharmaceutical composition according to claim 8 or 9, wherein the pharmaceutical composition is used in combination with at least one selected from the group consisting of an anti-PD-1 antibody, an anti-PD-L1 antibody, an anti-CTLA-4 antibody, and IL-2.

11. An agent for enhancing T-cell growth induction comprising the human IL-18 variant according to any one of claims 1 to 7.

12. A method for producing a genetically modified T cell, the method comprising a step of enhancing growth induction of a cell population including T cells in the presence of IL-2 and the human IL-18 variant according to any one of claims 1 to 7.

13. A human IL-18 variant having a G3L mutation relative to an amino acid sequence of wild-type human IL-18, wherein the human IL-18 variant optionally has an additional mutation of the following 1) or 2):
1) substitutions of cysteine at positions 38, 68, 76, and 127 with serine; or
2) substitutions of cysteine at positions 38, 68, 76, and 127 with serine and substitutions of glutamic acid at position 6 and lysine at position 53 with alanine.

14. A human IL-18 variant having a C38M mutation relative to an amino acid sequence of wild-type human IL-18, wherein the human IL-18 variant optionally has an additional mutation of the following 1) or 2):
1) substitutions of cysteine at positions 68, 76, and 127 with serine; or
2) substitutions of cysteine at positions 68, 76, and 127 with serine and substitutions of glutamic acid at position 6 and lysine at position 53 with alanine.
